# EUROPÄISCHE PATENTSCHRIFT

(11) **EP 3 787 563 B1**
(45) Veröffentlichungstag und Bekanntmachung des Hinweises auf die Patenterteilung: **11.03.2026**
(21) Anmeldenummer: 19720840.8
(22) Anmeldetag: 27.04.2019
(51) Int. Cl.: A61F 2/44

(54) **PLATZHALTER FÜR DIE WIRBELSÄULENCHIRURGIE**
PLACE HOLDER FOR SPINAL SURGERY
ÉLÉMENT D'ÉCARTEMENT POUR LA CHIRURGIE RACHIDIENNE

(30) Priorität: 01.05.2018 DE 102018206693; 30.01.2019 DE 102019201211
(43) Veröffentlichungstag der Anmeldung: 10.03.2021
(73) Patentinhaber: I-Pego GmbH, 19073 Wittenförden (DE)
(72) Erfinder: RIEGER, Bernhard, 32052 Herford (DE)
(74) Vertreter: Rößner, Ulrike
(86) Internationale Anmeldenummer: PCT/EP2019/060834
(87) Internationale Veröffentlichungsnummer: WO 2019/211208

(56) Entgegenhaltungen:
- US-A1- 2007 198 089
- US-A1- 2014 039 622
- US-A1- 2015 148 908
- US-A1- 2017 209 282
- US-A1- 2018 098 860

## Beschreibung

Die Erfindung betrifft einen Platzhalter für die Wirbelsäulenchirurgie, der ein oberes Auflager mit einer oberen Auflagefläche und ein unteres Auflager mit einer unteren Auflagefläche, deren relative Lage zueinander veränderbar ist, wobei die obere wie auch die untere Auflagefläche jeweils eine erste und ein zweite Teilfläche aufweist, die sich in einem geschlossenen Zustand des Platzhalters an einer Kante berühren, und eine Expansionsvorrichtung, mit der die Auflageflächen in ihrer lateralen Ausdehnung durch laterales Auseinanderdriften der ersten und zweiten Teilfläche zwischen einer minimalen lateralen Ausdehnung und einer maximalen lateralen Ausdehnung wie auch in ihrem vertikalen Abstand zwischen einer Minimalhöhe und einer Maximalhöhe des Platzhalters zueinander veränderbar sind, so dass der Platzhalter zwischen einem geschlossenen und einem expandiertem Zustand einstellbar ist, umfasst.

Die Degeneration von Wirbelsäulensegmenten der menschlichen Wirbelsäule ist Ursache für Beschwerden der Wirbelsäule, die häufig mit großen Schmerzen einhergehen. Sie ist ein ganz typisches Krankheitsbild, das eine große Zahl von Patienten, häufig im fortgeschrittenen Alter, betrifft und zu dauerhaften Beeinträchtigungen der Patienten führt. Dabei zeigt sie verschiedenste Erscheinungsformen. Zu den häufigsten Ausbildungen gehört zum einen die Degeneration der Bandscheibe, also der "Verschleiß" des knorpelartigen Gewebes der Bandscheibe, wodurch es zu Fehllagen kommt. Zum anderen gehört hierzu die Osteoporose, also die Zersetzung der Binnenstruktur (Spongiosa) des Knochens, insbesondere des Wirbels, wodurch dieser brüchig wird und es in Folge zu Deckplattenimpressionsfrakturen und zum Einsintern der Wirbelkörper kommt.

Die Behandlung erfolgt unter anderem operativ - durch Entnahme von mindestens Teilen eines degenerierten Wirbelsäulensegments und/oder der Implantation von platzhaltenden Implantaten an Stelle einer entnommenen Bandscheibe oder eines entnommenen Bandscheibensegments oder aber zur inneren Stützung durch Implantation in den Wirbelkörper oder anstelle eines Wirbelkörpers.

Um große Wunden und damit größere Schmerzen und hohen Blutverlust zu vermeiden, werden in der Chirurgie, so auch in der Wirbelsäulenchirurgie, wo immer möglich mikroskopische bzw. endoskopische Verfahren eingesetzt, um über eine kleine Wunde an den Ort des Geschehens zu kommen. Der direkte minimalinvasive chirurgische Zugang zur menschlichen Wirbelsäule von dorsal (also vom Rücken des Patienten aus) ist einfach, schnell und sicher. Um die Innervation der autochthonen Rückenmuskulatur durch den chirurgischen Zugangsweg nach Möglichkeit wenig zu schädigen, wird dabei der einseitige Zugangsweg präferiert.

Implantate sollten während ihrer Heranführung an ihren Wirkungsort in der Wirbelsäule eines Patienten kompakt und an den Zugangsweg angepasst, jedoch am Wirkungsort selbst "entfaltbar" bzw. auf dort erforderliche Größe expandierbar sein.

Solche platzhaltenden Implantate - bzw. Platzhalter - für die Wirbelsäulenchirurgie können eine Bandscheibe bzw. ein Bandscheibensegment ersetzen. Wenn der Platzhalter als Bandscheibenersatz eingesetzt wird, dient er als Zwischenwirbelimplantat zur interkorporellen Fusion von Wirbeln. Dieses Zwischenwirbelimplantat, das die häufigste Anwendungsform des hier beschriebenen Platzhalters darstellt und auch "Cage" genannt wird, muss an seinem Wirkungsort als Abstandshalter agieren und gleichzeitig eine stützende Wirkung ausüben - in diesem Fall zwischen zwei benachbarten Wirbelkörpern.

Prinzipiell ist der Einsatz von hier beschriebenen Platzhaltern auch zum vollständigen oder partiellen Ersatz eines Wirbelkörpers möglich, wobei der Platzhalter bei vollständigem Ersatz des Wirbelkörpers im Sinne eines Abstandhalters oder bei partiellem Ersatz im Sinne einer Augmentierung (also einer Wiederaufrichtung), einer Stützung bzw. einer Stabilisierung eines Wirbelkörpers im Wirbelkörper selbst zur Anwendung kommt (Spondyloplastie). Auch ein Platzhalter, der als vollständiger oder partieller Ersatz eines Wirbelkörpers verwendet wird, muss an seinem Wirkungsort als Abstandshalter agieren und gleichzeitig eine stützende Wirkung ausüben - in diesem Fall anstelle des Wirbelkörpers oder im Wirbelkörper selbst.

Es existieren Zwischenwirbelimplantate, also Platzhalter für die Wirbelsäulenchirurgie, die als Bandscheibenersatz genutzt werden, die in ihrer Höhe verändert werden können: Derartige Zwischenwirbelimplantate sind beispielsweise aus der DE 44 16 605 C1, der US 5,554,191 A oder der FR 2 719 763 A1 bekannt. Gemeinsam ist diesen Zwischenwirbelimplantaten, dass zwei Arme des Zwischenwirbelimplantats nach Ihrer Implantation durch Distanzelemente aufgespreizt werden, die in Längsrichtung der Arme zwischen diese eingeschoben werden und dabei an Aufgleitflächen, die als schiefe Ebenen in Form von planen Flächen ausgeführt sind, entlang gleiten, so dass abhängig von der Tiefe des Einschubes des Distanzelementes eine unterschiedliche Aufspreizung der Arme erfolgt. Dies hat einerseits einen veränderten Winkel der ehemals (also im geschlossenen Zustand) parallel zueinander verlaufenden Stützflächen der beiden Arme für die benachbarten Wirbelkörper zur Folge und führt gleichzeitig zu einer in asymmetrischer Form veränderten Höhe des Zwischenwirbelimplantats. Für die Verschiebung der Distanzelemente werden bei den bekannten Zwischenwirbelimplantaten dieser Art in der Regel in Bezug auf die Symmetrie des Zwischenwirbelimplantats zentral gelagerte Schraubspindeln verwendet.

Die Druckschriften US 2014/0039622 A1 und EP 2 777 630 A1 beschreiben hingegen Platzhalter für die Wirbelsäulenchirurgie, die in einer gleichzeitigen Bewegung lateral und vertikal expandierbar sind. Diese gleichzeitige Expansion in sowohl lateraler als auch vertikaler Richtung erfolgt durch ein Aufspreizen von oberen und unteren Auflagern mittels zweier gegenläufiger, vorzugsweise pyramidenförmiger Keile, die auf einer zentral gelagerten Schraubspindel verlaufen. Auch die Druckschrift US 2005/0124989 A1 beschreibt einen expandierbaren Platzhalter, der sich lateral und vertikal absolut gleichzeitig expandieren lässt.

Die US 2017/209282 A1 betrifft mit einem neurochirurgisches und orthopädisches Fixationssystem einen Platzhalter für die Wirbelsäulenchirurgie, mit oberem und unterem Auflager mit entsprechenden Auflageflächen und einer Expansionsvorrichtung, die die Auflageflächen in ihrer lateralen Ausdehnung und in ihrem vertikalen Abstand zwischen einer Minimalhöhe und einer Maximalhöhe verstellt. Das System ist so konzipiert, dass zunächst die laterale Ausdehnung abgeschlossen sein muss, damit die horizontale Expansion beginnen kann,

Die US 2018/098860 A1 beschreibt ein "intervertebrales Gerüstsystem", also einen Platzhalter für die Wirbelsäulenchirurgie, der einer externen Expansionsvorrichtung bedarf. Er enthält Stützstreben und Verbindungselemente zwischen den Stützstreben, die im geschlossenen Zustand gefaltet sind und unter Zuhilfenahme der externen Expansionsvorrichtung aufgefaltet werden, die hierzu ein Distanzelement in den Platzhalter einführt.

Heute eingesetzte Platzhalter für die Wirbelsäulenchirurgie sind also entweder lateral oder vertikal (also nur in eine Richtung) expandierbar. Andere heute eingesetzte Platzhalter mit einem einzigen Antrieb sind lateral und vertikal, jedoch nur gleichzeitig und proportional zueinander expandierbar. Eine dritte Gruppe von bislang beschriebenen Platzhaltern ist mittels getrennter Expansionsmechanismen, also gleich mit Hilfe zweier Antriebe oder mit zwei strikt aufeinander folgender Expansionsbewegungen, bei denen der Abschluss der ersten Bewegung die Voraussetzung für den Beginn der zweiten Bewegung ist, nacheinander expandierbar. Diese sind aber technisch nur sehr aufwändig herstellbar oder sind so konstruiert, dass sie keine Auflagefläche bieten.

Nur in eine Richtung expandierbare, beispielsweise nur vertikal expandierbare, Platzhalter sind dabei entweder zu groß für einen wirklich minimalinvasiven Eingriff, wenn sie - als Zwischenwirbelimplantat genutzt - von vornherein die laterale Ausdehnung aufweisen, die für die Stützung der Wirbelkörpergrundplatte des oberen Wirbelkörpers und der Wirbelkörperdeckplatte des unteren Wirbelkörpers nötig sind, um einen Einbruch in diese zu vermeiden. Sind nur in eine Richtung expandierbare, also beispielsweise nur vertikal expandierbare, Platzhalter hingegen so kompakt wie für ihre Heranführung an ihren Wirkungsort mittels minimalinvasiver Chirurgie nötig, so unterstützen sie während ihrer vertikalen Expansion nur Teilflächen der Wirbelkörpergrundplatte und der Wirbelkörperdeckplatte und können damit leicht in diese einbrechen. Bei Nutzung als vollständiger oder partieller Wirbelkörperersatz besteht die Gefahr prinzipiell ebenfalls - hier von der anderen Seite der Wirbelkörpergrundplatte bzw. der Wirbelkörperdeckplatte kommend -, wenn auch bei Weitem nicht so ausgeprägt.

Auch als Zwischenwirbelimplantat genutzte Platzhalter nach dem Stand der Technik, die gleichzeitig in beide Richtungen expandierbar sind, bannen diese Gefahr nicht, da sie von Anfang an während ihrer lateralen Expansion auch schon vertikal expandieren und damit schon eine große stützende Wirkung ausüben müssen, während sie noch gar nicht ihre vollständige lateral notwendige Ausdehnung erreicht haben, um dies auch ohne Gefahr eines Einbruchs in die Wirbelkörpergrundplatte und/oder die Wirbelkörperdeckplatte tun zu können. Zudem werden bei heute üblichen Platzhaltern, die lateral und vertikal gleichzeitig expandierbar sind, die oberen und unteren Auflager so in zwei Teilstücke aufgespreizt, dass im Mittelbereich wiederum eine nichtunterstützte Fläche entsteht, und die jeweiligen aufgespreizten Teilstücke in den Randbereichen der Wirbelkörpergrundplatte bzw. der Wirbelkörperdeckplatte einbrechen, da sie nun nur hier sehr lokal Stützfunktion ausüben.

Ein Platzhalter wie in der Druckschrift DE 10 2017 211 185 A1 beschrieben, der zwar über eine Schraubspindel aber mittels getrennter Expansionsmechanismen lateral und vertikal nacheinander expandierbar ist, ist als partieller oder vollständiger Wirbelkörperersatz wie in der Druckschrift beschrieben einsetzbar, bietet jedoch als Zwischenwirbelimplantat anstelle der Bandscheibe bzw. des Bandscheibensegments nicht das notwendige Auflager.

Alle im Stand der Technik beschriebenen Platzhalter sind bezüglich ihrer Form auf einen geraden Implantations- bzw. Zugangsweg ausgerichtet und sind für nicht gerade Implantations- bzw. Zugangswege "sperrig". Wird der Platzhalter jedoch als Zwischenwirbelimplantat genutzt, so sollte er idealerweise von dorsalmedian oder paramedian eingebracht werden können und symmetrisch neutral des Duralschlauchs (von etwa dem zweiten Lendenwirbel nach distal) oder des Rückenmarks (bis etwa zum zweiten Lendenwirbel), also ventral des Wirbelkanals zu liegen kommen, ohne jedoch den Wirbelkanal zu passieren, um die darin enthaltenen neurologischen Strukturen nicht zu schädigen.

Der vorliegenden Erfindung liegt daher die Aufgabe zugrunde, einen Platzhalter für die Wirbelsäulenchirurgie der eingangs genannten Art anzugeben und weiterzubilden, durch welchen die vorgenannten Probleme überwunden werden. Insbesondere soll ein Platzhalter für die Wirbelsäulenchirurgie beschrieben werden, der kompakt und minimalinvasiv in entsprechenden Stabilisierungsverfahren, wie beispielsweise in der minimalinvasiven Vektor-Lumbalen-Interkorporellen Fusion (MIS-VLIF), Anwendung findet und dabei vorzugsweise in einfacher Weise auch über einen nicht geraden Implantationsweg implantierbar ist, der am Wirkungsort die notwendige laterale Auflagefläche entwickeln kann, um seiner Stützfunktion gerecht zu werden, und mit dem ein gewünschter vertikaler Abstand, wie der vertikale Abstand zwischen zwei Wirbelkörpern, sicher und ohne Einbruchrisiko eingestellt werden kann. Der Platzhalter soll zudem den biokinemetrischen Erfordernissen seines Einsatzes Rechnung tragen, und eine natürliche Funktion der Wirbelsäule bestmöglich unterstützen, indem er die funktionelle Anatomie des zu stabilisierenden Bewegungsabschnittes berücksichtigt.

Zudem sollen entsprechende Verfahren zur Implantation des Platzhalters in der Wirbelsäule eines Patienten angegeben werden.

Die Aufgabe wird erfindungsgemäß durch die Lehre des Patentanspruchs 1 gelöst. Weitere vorteilhafte Ausgestaltungen und Weiterbildungen der Erfindung gehen aus den Unteransprüchen hervor.

Ein Platzhalter für die Wirbelsäulenchirurgie umfasst ein oberes Auflager mit einer oberen Auflagefläche und ein unteres Auflager mit einer unteren Auflagefläche, deren relative Lage zueinander veränderbar ist. Sowohl die obere als auch die untere Auflagefläche weist jeweils eine erste und ein zweite Teilfläche auf, die sich in einem geschlossenen Zustand des Platzhalters an einer Kante berühren.

"Jeweils eine erste und eine zweite Teilfläche" ist als "jeweils mindestens eine erste und eine zweite Teilfläche" zu lesen. Prinzipiell ist auch eine weitere Aufteilung der Auflageflächen möglich: Die erste wie auch die zweite Teilfläche wäre beispielsweise in einer speziellen Ausführungsform nochmals unterteilbar in jeweils zwei Unterflächen, was dann zu vier Teilflächen der oberen Auflagefläche bzw. der unteren Auflagefläche führt. Allerdings führt eine Aufteilung der oberen bzw. der unteren Auflagefläche in jeweils mehr als zwei Teilflächen zu einem ggf. komplizierteren inneren Aufbau des Platzhalters.

Der geschlossene Zustand des Platzhalters ist derjenige, in der seine laterale wie auch seine vertikale Ausdehnung einen Minimalwert aufweist. In diesem Zustand - also so klein bzw. kompakt wie möglich -, der der Insertionszustand des Platzhalters ist, wird der Platzhalter an seine Position im Wirbelsäulenbereich durch eine minimale Öffnung im Rückenbereich des Patienten eingeführt.

In diesem geschlossenen Zustand befinden sich oberes und unteres Auflager in einem Minimalabstand zueinander. Gleiches gilt für die erste und zweite Teilfläche der oberen bzw. unteren Auflagefläche, die sich im geschlossenen Zustand in der Regel entlang einer Kante berühren, da dies eine kleinstmögliche laterale Ausdehnung des Platzhalters unterstützt. Dabei ist es jedoch nicht erforderlich, dass tatsächlich eine körperliche Berührung der beiden Kanten vorliegt: Von dem Begriff "berühren" soll folglich auch ein Nebeneinanderliegen der beiden Kanten mit einem sehr geringen Abstand, typischerweise von kleiner 1mm, maximal von kleiner 2mm umfasst sein. Die Kante, an der sich die beiden Teilflächen berühren (bzw. entlang derer sie mit sehr geringem Abstand nebeneinander liegen) muss dabei keine Gerade sein, sondern kann jegliche Form annehmen.

Der Platzhalter für die Wirbelsäulenchirurgie umfasst des Weiteren eine Expansionsvorrichtung, mit der die Auflageflächen in ihrer lateralen Ausdehnung durch laterales Auseinanderdriften der ersten und zweiten Teilfläche der oberen und der unteren Auflagefläche bis zu einem Maximaldriftbetrag zwischen einer minimalen lateralen Ausdehnung und einer maximalen lateralen Ausdehnung, wie auch in ihrem vertikalen Abstand der oberen und der unteren Auflagefläche zwischen einer Minimalhöhe und einer Maximalhöhe des Platzhalters (da die obere und untere Auflagefläche zugleich die obere und die untere Begrenzung des Platzhalters darstellt) zueinander veränderbar sind, so dass der Platzhalter zwischen einem geschlossenen und einem expandiertem Zustand einstellbar ist. Dabei verläuft die vertikale Richtung nach Implantation in die Wirbelsäule des Patienten in etwa parallel der Wirbelsäule bzw. parallel einer von caudal nach cranial verlaufenden Richtung. Die laterale Bewegung der Teilflächen erfolgt hingegen senkrecht hierzu, also in der Horizontalen.

Um eine Expansion in einfachster Art und Weise nach dem Verbringen des Platzhalters an seinen Wirkungsort in der Wirbelsäule des Patienten zu realisieren, enthält die Expansionsvorrichtung einen einzigen Antrieb, d.h., die Durchführung der Expansion, und damit sowohl der Veränderung der lateralen Ausdehnung der Auflageflächen als auch die Veränderung des vertikalen Abstands der Auflageflächen wird über ein und denselben Antrieb realisiert: Der Chirurg bedient hierfür beispielsweise in linearer Form immer dasselbe Stellelement, das wiederum einen Mechanismus zur Expansion bedient, der nicht oder nicht durchgängig linear verlaufen muss.

Wesentlich komplizierter im Aufbau, aber dennoch denkbar, ist eine Expansionsvorrichtung mit voneinander getrennten Antrieben für die Veränderung der lateralen Ausdehnung und der Veränderung des vertikalen Abstands.

Die Expansionsvorrichtung verändert also sowohl die relative Lage der oberen Auflagefläche zur unteren Auflagefläche, wie auch die laterale Ausdehnung der oberen und der unteren Auflagefläche. Der Begriff "expandierter Zustand" ohne jeglichen Zusatz kennzeichnet dabei den maximal expandierten Zustand. Es kann durchaus gewünscht oder vorteilhaft sein, einen teilweise expandierten Zustand einzustellen: Beispielsweise, wenn nach der Implantation des Platzhalters dieser zunächst nicht den vollen vertikalen Abstand und/oder die Auflagefläche nicht die volle laterale Ausdehnung aufweisen soll, kann in einer speziellen Ausführungsform ein teilweise expandierter Zustand eingestellt werden. Wiederum vorteilhafterweise ist jeder dieser teilweise expandierten Zustände fixierbar, ggf. wird ein "Zurückgleiten" bzw. "Wiederzusammenfalten" aber auch schon durch eine hohe Selbsthemmung der Expansionsvorrichtung, wie z.B. eines dort eingesetzten Gewindes, erreicht.

Eine Einstellbarkeit des Zustands zwischen einem geschlossenen Zustand und einem expandierten Zustand erfolgt i.d.R. kontinuierlich, insbesondere, wenn keine teilweise expandierten Zustände über einen längeren Zeitraum gehalten werden sollen. Eine solche kontinuierliche Einstellbarkeit bzw. kontinuierlichen Veränderung der lateralen Ausdehnung und des vertikalen Abstands ist vorteilhaft, aber nicht zwingend.

Des Weiteren ist der erfindungsgemäße Platzhalter bevorzugt auch von einem expandierten

Zustand in einen geschlossenen Zustand - auch Insertionszustand genannt - zurückstellbar und erlaubt somit einen einfachen Austausch im Falle der Unzufriedenheit des Chirurgen oder des Patienten mit dem Ergebnis.

Erfindungsgemäß ist nun der Platzhalter dadurch gekennzeichnet, dass die Expansionsvorrichtung eingerichtet ist, die Veränderung der lateralen Ausdehnung und des vertikalen Abstands in zwei voneinander unabhängig und frei definierbaren und im Platzhalter kodierten Bewegungsverläufen mittels eines einzigen Antriebs auszuführen.

In dem erfindungsgemäßen Platzhalter ist also in ein und derselben Expansionsvorrichtung der Verlauf der Veränderung der lateralen Ausdehnung und der Verlauf der Veränderung des vertikalen Abstands im Verlaufe seiner Expansion - durch ein und denselben konzeptionellen Aufbau - frei definierbar kodiert. Dies bedeutet, dass für jede laterale Ausdehnung der oberen und unteren Auflageflächen zwischen der minimalen und der maximalen lateralen Ausdehnung ein dazu gewünschter vertikaler Abstand der oberen und unteren Auflageflächen voneinander im Rahmen eines Betrags zwischen einer Minimalhöhe und einer Maximalhöhe des Platzhalters einstellbar ist.

Damit muss weder das Verhältnis zwischen der Veränderung der lateralen Ausdehnung und der Veränderung des vertikalen Abstands konstant gehalten werden, noch müssen die Bewegungen nacheinander oder durch getrennte Expansionsmechanismen bzw. - vorrichtungen ausgeführt werden.

Vorzugsweise umfasst der erfindungsgemäße Platzhalter ein physisches Abbild eines Ablaufplans der Veränderung der lateralen Ausdehnung zur Veränderung des vertikalen Abstands im Verlaufe seiner Expansion. Dies kann beispielsweise seinen Ausdruck finden in der Form von Freiformflächen im Inneren bzw. an den Innenseiten oder Kanten des oberen wie auch des unteren Auflagers oder aber beispielsweise auf hierfür verwendeten Distanzelementen, die als Gleitflächen genutzt werden, in der (frei bestimmbaren) Form von als Gleitlöcher eingesetzten Langlöchern, oder aber in drehbaren Strukturen mit unterschiedlicher Zahnteilung.

Vorteilhafterweise wird sich in einem erfindungsgemäßen Platzhalter das Verhältnis von Veränderung der lateralen Ausdehnung zur Veränderung des vertikalen Abstands im Verlaufe seiner Expansion mehrfach abschnittsweise oder aber kontinuierlich ändern. Dabei wird vorzugsweise die Veränderung der lateralen Ausdehnung und des vertikalen Abstands zumindest teilweise zeitlich voneinander getrennt, derart, dass nach seiner Einführung, beispielsweise in einen Bandscheibenraum der Wirbelsäule eines Patienten, in dem die Bandscheibe vorher entfernt wurde, der im geschlossenen Zustand eingeführte Platzhalter zunächst eine laterale Ausdehnung erfährt, um eine ausreichend breite Auflagefläche für die dann etwas später parallel einsetzende Veränderung des vertikalen Abstands zwischen den beiden Wirbelkörpern zu bieten.

Dabei ist beispielsweise eine laterale Expansion im Verhältnis zum linearen Antrieb als eine überproportionale Ausdehnung, und damit als eine nicht lineare Vergrößerung, der wirksamen Auflageflächen beschreibbar, bei der zunächst große Veränderungen der lateralen Ausdehnung vollzogen werden, während eine mittendrin einsetzende vertikalen Expansion und damit Änderung des vertikalen Abstands dann linear verläuft oder umgekehrt.

Denkbar ist auch, dass die laterale Expansion und die vertikale Expansion überproportional und voneinander unabhängig über einen gemeinsamen linearen Antrieb realisiert werden.

Die Art und Weise der Veränderung der lateralen Ausdehnung der Auflageflächen zur Veränderung ihres vertikalen Abstands kann also ggf. den Nutzungen, den spezifischen Problemen des Patienten aber auch den Wünschen des behandelnden Chirurgen angepasst werden. Dabei kann eine Serie von Platzhaltermodellen geschaffen werden, die häufig genutzte Konstellationen abbilden, alle nach demselben Prinzip arbeiten, aber sich beispielsweise in der Formung der Freiformflächen und/oder der Formung der als Gleitlöcher eingesetzten Langlöcher unterscheiden.

Die Nutzung von Freiformflächen und/oder als Gleitlöchern genutzten Langlöchern bietet zudem die Möglichkeit, bestimmte Zustände auf einfache Weise dadurch zu fixieren oder zu stabilisieren, dass mittels der Formung der Freiformflächen und/oder der als Gleitlöcher genutzten Langlöcher der vertikale Abstand kurzzeitig wieder verringert wird und damit ein metastabiler Zustand erreicht wird.

Des Weiteren ist der erfindungsgemäße Platzhalter bevorzugt auch von einem expandierten Zustand in einen geschlossenen Zustand - auch Insertionszustand genannt - zurückstellbar, die laterale Ausdehnung der oberen und unteren Auflageflächen können also von einer maximalen Ausdehnung zurück in eine minimale Ausdehnung und der vertikale Abstand der oberen und unteren Auflageflächen von einer Maximalhöhe zurück auf eine Minimalhöhe gestellt werden, indem der Antrieb der Expansionsvorrichtung in entgegengesetzte Richtung betrieben wird.

In einer bevorzugten Ausführungsform weisen die erste wie auch die zweite Teilfläche der oberen wie auch der unteren Auflagefläche an der Kante, an der sie sich im geschlossenen Zustand berühren, eine ineinandergreifende Struktur auf, die derart gestaltet ist, dass sie bei einer Expansion ein laterales Auseinanderdriften der ersten und zweiten Teilfläche ermöglicht, und im expandierten Zustand ein senkrecht zur Richtung des lateralen Auseinanderdriftens durch die oberen wie auch die untere Auflagefläche verlaufender lateraler Spalt eine Spaltbreite aufweist, die kleiner ist als der Maximaldriftbetrag.

Die ineinandergreifende Struktur der ersten Teilfläche der oberen Auflagefläche ist dabei auf die ineinandergreifende Struktur der zweiten Teilfläche der oberen Auflagefläche abgestimmt: Die ineinandergreifende Struktur der ersten und der zweiten Teilflächen sollen also im geschlossenen Zustand ineinander fassen.

Vorzugsweise greifen sie möglichst passgenau ineinander - nach einem Schlüssel-Schloss-Prinzip. Gleiches gilt für die ineinandergreifende Struktur der ersten Teilfläche der unteren Auflagefläche und die ineinandergreifende Struktur der zweiten Teilfläche der unteren Auflagefläche. Jedoch müssen die entsprechenden Strukturen der Teilflächen der oberen Auflagefläche und der unteren Auflagefläche nicht identisch oder ähnlich sein. Identische bzw. ähnliche Strukturen sind jedoch vorteilhaft, da dann das Expansionsverhalten der oberen Auflagefläche an der Wirbelkörpergrundplatte des oberen Wirbelkörpers und der unteren Auflagefläche an der Wirbelkörperdeckplatte des unteren Wirbelkörpers bei einer Nutzung des Platzhalters als Zwischenwirbelimplantat identisch bzw. ähnlich ist.

Um ein laterales Auseinanderdriften - also ein laterales Gleiten in entgegengesetzte Richtung - der ersten und zweiten Teilfläche zu ermöglichen (und eben nicht zu behindern) müssen die ineinandergreifenden Strukturen so ausgebildet sein, dass sie sich beim lateralen Auseinanderdriften nicht ineinander verhaken können. Ideale Strukturformen solcher ineinandergreifenden Strukturen sind beispielsweise eine Zahnstruktur, eine ZickZack-Struktur oder eine gewellte Struktur, wobei keine dieser Strukturen regelmäßig ausgeformt sein muss. Die Ausformung einer "idealen" ineinandergreifenden Struktur ist letztlich davon abhängig, welcher Abbildungsvorschrift das laterale Auseinanderdriften gehorcht.

Das laterale Auseinanderdriften erfolgt dabei bevorzugt durch ein aneinander Auseinandergleiten: Bei einer Zahnstruktur beispielsweise heißt dies, dass die Bereiche der ineinandergreifenden Struktur der Kanten der ersten und zweiten Teilfläche in den parallel zur lateralen Driftrichtung ausgerichteten Bereichen der Kanten aneinander auseinander gleiten, was den Halt, den die Auflagefläche auch beim Auseinanderdriften weiterhin bietet, zusätzliche verstärkt.

Die Spaltbreite eines lateralen Spalts, der beim Auseinanderdriften entsteht, ist dabei kleiner bzw. idealerweise wesentlich kleiner als der Maximaldriftbetrag. Der laterale Spalt ist dabei der "gedachte" Spalt, der zwischen der ersten Teilfläche und der zweiten Teilfläche der oberen Auflagefläche bzw. der unteren Auflagefläche entlang der gesamten Länge der Auflagefläche senkrecht zur lateralen Driftrichtung entstünde, wenn man gerade Kanten hineinlegen würde, die entlang der im weitesten "vorstehenden" Bereiche der ineinandergreifenden Struktur verlaufen.

Wenn der Platzhalter als Zwischenwirbelkörperimplantat eingesetzt wird, dann übt er Druck aus bzw. unterstützt den darüber wie auch darunter befindlichen Wirbelkörper anstelle der Bandscheibe. Während mit gerade verlaufenden Kanten der ersten und zweiten Teilfläche beim Auseinanderdriften vom Anfang an ein solcher lateraler Spalt entsteht, dessen Spaltbreite schnell einen Wert annimmt, bei dem eine Unterstützung der Wirbelkörpergrundplatte des oberen Wirbelkörpers und der Wirbelkörperdeckplatte des unteren Wirbelkörpers im zentralen Bereich nicht mehr gewährleistet werden kann, bieten erfindungsgemäße ineinandergreifende Strukturen eine ausreichende Stützfunktion auch bei ihrer maximalen lateralen Ausdehnung. Die erfindungsgemäßen ineinandergreifenden Strukturen solcher Platzhalter erhöhen also die effektive Wirkung der Auflageflächen im expandierten Zustand signifikant gegenüber Platzhaltern nach dem Stand der Technik.

Die anstelle eines durchgehenden lateralen Spalts entstehenden kleineren Öffnungen lassen eine knöcherne Durchbauung zu und unterstützen diese durch eine Vergrößerung der Oberfläche, an der entlang eine solche Durchbauung stattfinden kann. Dadurch wird das Interface zwischen Knochen und Implantat verbessert, was das Einheilen des Implantats bzw. die Integration eines solchen Platzhalters in die Wirbelsäule unterstützt.

Die Hauptnutzung des hier beschriebenen Platzhalters betrifft seinen Einsatz als Zwischenwirbelkörperimplantat, auch Cage bzw. interkorporeller Abstandhalter genannt, um anstelle der Bandscheibe oder aber anstelle von Teilbereichen der Bandscheibe implantiert zu werden: Hierfür sind die erfindungsgemäßen Eigenschaften der Auflageflächen dieses Platzhalters am wichtigsten.

Nichtsdestotrotz sind weitere Nutzungen des erfindungsgemäßen Platzhalters möglich als partieller oder kompletter Wirbelkörperersatz oder auch zur Aussteifung eines Wirbelkörpers mit einem Knochenfüllstoff im Sinne einer Spondyloplastie: Dabei wird der einstellbare Platzhalter nach Eröffnung der kortikalen Knochenstruktur in den Wirbelkörper zur Aussteifung des Wirbelkörpers bei Frakturen oder bei der Osteoporose implantiert und dann verfüllt.

In einer bevorzugten Ausführungsform des erfindungsgemäßen Platzhalters beträgt die Spaltbreite des lateralen Spalts auch im expandierten Zustand Null. Dies ist sehr leicht erreichbar bei Nutzung einer Zahnstruktur, wie in den Figuren leicht nachvollziehbar ist. Allerdings sind viele weiteren Strukturformen denkbar, die dazu führen, dass auch im expandierten Zustand die Spaltbreite des lateralen Spalts gleich Null ist: Eine Wellenform mit einer Amplitude, die größer ist als der halbe Maximaldriftbetrag, erfüllt beispielsweise ebenfalls die Bedingung.

In einer besonders bevorzugten Ausgestaltung ist das obere wie auch das untere Auflager des Platzhalters derart geformt, dass beim lateralen Auseinanderdriften die ineinandergreifende Struktur der ersten Teilfläche auf einer Auflagestruktur im zur zweiten Teilfläche zugehörigen Teil des Auflagers und die ineinandergreifende Struktur der zweiten Teilfläche auf einer Auflagestruktur im zur ersten Teilfläche zugehörigen Teil des Auflagers lagernd gleitet. Dies verschafft den Auflageflächen des Platzhalters bei ihrer lateralen Expansion zusätzliche Stabilität.

Mit den erfindungsgemäßen Platzhalter mit durch die oberen wie auch die untere Auflagefläche verlaufendem lateralen Spalt mit einer Spaltbreite, die (deutlich) kleiner ist als der Maximaldriftbetrag der lateralen Ausdehnung dieser Auflageflächen und mit einer Expansionsvorrichtung, die die Veränderung der laterale Ausdehnung und des vertikalen Abstands in zwei voneinander unabhängig und frei definierbaren Bewegungsverläufen auszuführen vermag, ist ein minimalinvasiv implantierbarer Platzhalter beschrieben, der am Wirkungsort die notwendige Größe der Auflagefläche entwickeln kann, um seiner Stützfunktion gerecht zu werden, und mit dem ein gewünschter vertikaler Abstand, wie der vertikale Abstand zwischen zwei Wirbelkörpern, sicher und ohne Einbruchrisiko eingestellt werden kann. Dabei trägt er den biokinemetrischen Erfordernissen seines Einsatzes Rechnung und unterstützt eine natürliche Funktion der Wirbelsäule bestmöglich, indem er die sagittale Balance verbessert.

Er bietet ein deutlich größeres wirksames Auflager als Platzhalter nach dem Stand der Technik, die dorsal eingebracht werden, insbesondere eine deutlich funktioneller verteilte Auflagefläche mit verbessertem Footprint und optimalem Press-fit (also eine bessere "Verankerung" in der Wirbelsäule aufgrund seiner Form und des ausgeübten Drucks und seiner Verteilung). Der erfindungsgemäße Platzhalter berücksichtigt dabei das biologisch ableitbare optimale Expansionsverhalten durch einen frei definierbaren Expansionsverlauf der Veränderung der lateralen Ausdehnung, also seiner Expansion in die Breite, die nach dem Verbringen des Platzhalters an seinen Wirkungsort vorzugsweise zuerst gestartet wird, und der Veränderung des vertikalen Abstands, also seiner Expansion in die Höhe, die vorzugsweise etwas verzögert oder nachfolgend eingeleitet wird, obgleich dies mit demselben Antrieb bedient wird. Der erfindungsgemäße Platzhalter vereint dabei mehrere von einem Platzhalter zur Implantation in der Wirbelsäule prinzipiell geforderten Eigenschaften.

Wesen der Erfindung ist also auch ein Expansionsmechanismus, der die laterale und die vertikale Expansion voneinander unabhängig gestaltbar macht, trotzdem er nur über einen Antrieb zur Realisierung der Expansion verfügt. Der Expansionsmechanismus des Platzhalters lässt es zu, dass die beiden Expansionsqualitäten in ihrer Abfolge und in jedweder belieben Verhältnismäßigkeit zueinander technisch in dem Platzhalter niedergelegt bzw. kodiert werden können.

In einer bevorzugten Ausgestaltung des Platzhalters bzw. der oben beschriebenen Platzhalter enthält die Expansionsvorrichtung zur Einstellung des Platzhalters zwischen dem geschlossenen und dem expandierten Zustand eine Schraubspindel mit einem Schraubenkopf, mit der die Veränderung der lateralen Ausdehnung wie auch des vertikalen Abstands steuerbar ist.

Die Schraubspindel ist in dieser Ausgestaltung also das wesentliche Element des Antriebs der Expansionsvorrichtung. Über ihren Schraubenkopf kann mit einem die minimalinvarsive Chirurgie unterstützendem Werkzeug auf die Schraubspindel zugegriffen werden. Der Schraubenkopf kann hierzu über eine beliebige Form und Ausgestaltung verfügen, die es erlaubt, mit dem von außen eingeführtem Werkzeug anzugreifen, um eine Drehbewegung der Schraubspindel zu erreichen und auch entsprechend steuern zu können, um damit wiederum die Veränderung der lateralen Ausdehnung wie auch des vertikalen Abstands zu steuern.

Die Achse der Schraubspindel muss dabei nicht zentral bzw. mittig im Platzhalter verlaufen. Sie kann vielmehr eine beliebige Lage, vorzugsweise senkrecht der Richtung der lateralen Ausdehnung, einnehmen. Dabei kann sie so platziert werden, dass der Schraubenkopf der Schraubspindel nach der Einführung des Platzhalters an seinen Wirkungsort leichter erreichbar ist als für Platzhalter nach dem Stand der Technik, die eine mittige Lage eines solchen Antriebselements erfordern. In dieser Ausgestaltung wandert die Schraubspindel des Antriebs im Sinne einer sich in ihrer Position veränderten Drehachse, also als technisch realisierte Centrode bzw. Gangpolbahn.

Mittels der Schraubspindel kann in einfachster Art und Weise die Expansion des Platzhalters ausgeführt werden, wenn sie wiederum Teil eines Keilmechanismus oder auf dem Prinzip von Keil und Gegenkeil beruhendem Mechanismus ist, mit dem die Veränderung der lateralen Ausdehnung wie auch des vertikalen Abstands der Auflageflächen gleichzeitig betrieben wird.

In wesentlich vorteilhafteren Ausgestaltungen jedoch ermöglicht die Drehung der Schraubspindel eine Expansion des Platzhalters derart, dass die Veränderung der lateralen Ausdehnung und des vertikalen Abstands in zwei
voneinander unabhängig und frei definierbaren Bewegungsverläufen erfolgt. Insbesondere ermöglicht sie in vorteilhafter Ausführung die zumindest
teilweise oder vollständige Trennung der Veränderung der lateralen Ausdehnung und des vertikalen Abstands voneinander im Hinblick auf ihre aufeinander bezogene Abfolge.

In einer speziellen Ausgestaltung des Platzhalters weist dieser in einer beliebigen lateralen Schnittebene keine Symmetrien entlang einer Achse, die parallel der Achse der Schraubspindel verläuft, auf. Ein paralleler Verlauf beinhaltet dabei auch eine Lage auf der Achse der Schraubspindel.

In einer besonders bevorzugten Ausgestaltung des erfindungsgemäßen Platzhalters umfasst dessen Expansionsvorrichtung des Weiteren ein bewegliches Distanzelement. Ein solches Distanzelement beabstandet zwei bewegliche Teile derart, dass eine Relativbewegung der beiden Teile zueinander nur entlang vorgegebener Bahnen oder nur in vorgegebenen Bereichen stattfinden kann. Solch ein Distanzelement kann ein Nocken sein bzw. eine nockenartige Gestalt aufweisen, aber auch andere, speziellere geometrische Formen annehmen. Wichtig ist jedoch die bewegliche Lagerung des Distanz-elements in jedem der beiden beweglichen Teile.

Dieses Distanzelement ist in diesem Fall nun an einer Position auf dem Distanzelement, vorzugsweise an einem ersten Ende, lateral drehbar um eine bewegliche Achse gelagert, die mittels einer in die bewegliche Achse integrierte und auf einem Gewinde der Schraubspindel (das demnach ein Außengewinde ist) laufenden Gewindemutter entlang der Schraubspindel verschiebbar ist, wobei die bewegliche Achse direkt oder indirekt in einem oberen Langloch in der ersten Teilfläche des oberen Auflagers und in einem unterem Langloch in der ersten Teilfläche des unteren Auflagers laufend angeordnet ist.

"Direkt in einem Langloch angeordnet" heißt dabei, dass die Achse direkt in dem Langloch verläuft, "indirekt in einem Langloch angeordnet" bedeutet, dass ein mit der Achse verbundenes Element in dem Langloch verläuft.

Das Distanzelement ist des Weiteren lateral drehbar gelagert um eine fixe Achse, die in der zweiten Teilfläche des oberen Auflagers und in der zweiten Teilfläche des unteren Auflagers lateral ortsunveränderlich gelagert ist. Diese fixe Achse befindet sich vorzugsweise in seinem zentralen Bereich. Es ist jedoch auch eine Anordnung der fixen Achse an einem zweiten Ende des Distanzelements möglich. Auch besteht die Möglichkeit der Realisierung mit einer nicht fixen Achse.

Und schließlich ist das Distanzelement an einem ersten und/oder einem zweiten Ende frei gleitend auf einer oberen dreidimensionalen Gleitfläche unter der ersten und/oder der zweiten Teilfläche des oberen Auflagers sowie auf einer unteren dreidimensionalen Gleitfläche über der ersten und/oder zweiten Teilfläche des unteren Auflagers gelagert, wobei die obere dreidimensionale Gleitfläche und die untere dreidimensionale Gleitfläche so zueinander geformt sind, dass das erste und/oder zweite Ende des Distanzelements für jeden Drehwinkel des Distanzelements um die fixe Achse eine definierte Position auf der oberen wie auch auf der unteren dreidimensionalen Gleitfläche einnimmt, deren absolute Lage und die dazu korrespondierende Position der beweglichen Achse in den entsprechend geformten Langlöchern die laterale Ausdehnung des Platzhalters und deren Abstand der oberen und der unteren dreidimensionalen Gleitfläche zueinander im geschlossenen Zustand die Höhe des Platzhalters bestimmt.

Eine dreidimensionale Gleitfläche ist eine im dreidimensionalen Raum ausgebildete Fläche, die - wie oben bereits erläutert - in bevorzugter Ausführungsform frei derart geformt wird, dass sie bzw. dass sie in Kombination mit einer weiteren dreidimensionalen Gleitfläche und/oder der Form des als Gleitlochgenutzten Langlochs den lateralen und vertikalen Bewegungsverlauf abbildet.

In einer weiteren besonders bevorzugten Ausgestaltung des erfindungsgemäßen Platzhalters umfasst dessen Expansionsvorrichtung ein bewegliches Distanzelement, das entlang der Schraubspindel bewegbar ist und (mindestens) vier Führungselemente aufweist, die jeweils in einem ersten oberen Langloch und/oder unter einer ersten oberen Führungsgleitfläche in der ersten Teilfläche und in einem zweiten oberen Langloch und/oder unter einer zweiten oberen Führungsgleitfläche der zweiten Teilfläche des oberen Auflagers und in einem ersten unteren Langloch und/oder auf einer ersten unteren Führungsgleitfläche der ersten Teilfläche und in einem zweiten unteren Langloch und/oder auf einer zweiten unteren Führungsgleitfläche der zweiten Teilfläche des unteren Auflagers verschiebbar sind. In dieser Ausgestaltung weist das Distanzelement selbst obere und untere bevorzugt dreidimensionale Gleitflächen auf. Dabei sind die obere Gleitfläche und die untere Gleitfläche auf dem Distanzelement so zueinander geformt, und die Langlöcher und/oder Führungsgleitflächen in jeder der Teilflächen des oberen und unteren Auflagers so geformt und angeordnet, dass für eine definierte Position, die das Distanzelement auf der Schraubspindel einnimmt, die dazu korrespondierende Position der Führungselemente in den entsprechend geformten Langlöchern und/oder Führungsgleitflächen die laterale Ausdehnung des Platzhalters und deren Abstand der oberen und der unteren Gleitfläche zueinander auf dem Distanzelement an einer Berührungskante und/oder an einer der Position des Distanzelements zugeordneten Position der oberen und unteren Führungsgleitflächen des oberen und des unteren Auflagers die Höhe des Platzhalters bestimmt. Führungsgleitflächen sind dabei spezielle Gleitflächen, die so ausgestaltet sind, dass sie neben der vertikalen Positionierung (und damit den Grad der vertikalen Expansion des Platzhalters) auch die laterale Positionierung (und damit den Grad der lateralen Expansion des Platzhalters) übernehmen.

Vorzugsweise ermöglichen dabei auch hier Formelemente auf der oberen und/oder der unteren dreidimensionalen Gleitfläche und/oder die Formung des Langlochs eine relative Fixierung spezieller, vorbestimmter Positionen.

Des Weiteren ist hier in einer Variante dieser Ausgestaltung des erfindungsgemäßen Platzhalters für die Veränderung des vertikalen Abstands eine Kombination der Formung der Gleitflächen auf dem Distanzelement und einer Formung auf einer Innenseite des oberen und unteren Auflagers möglich.

In einer dritten besonders bevorzugten Ausgestaltung des erfindungsgemäßen Platzhalters umfasst dessen Expansionsvorrichtung des Weiteren ein bewegliches Doppelpaar von Distanzelementen, das an einer Position auf jedem Distanzelement des Doppelpaars jeweils lateral drehbar um eine bewegliche Achse gelagert ist, wobei die beweglichen Achsen auf einer Gewindemutter angeordnet sind, und mittels der auf einem Gewinde der Schraubspindel laufenden Gewindemutter entlang der Schraubspindel verschiebbar sind. Des Weiteren ist das erste Distanzelement des Doppelpaars lateral drehbar um eine fixe Achse, die in der ersten Teilfläche des oberen Auflagers und in der ersten Teilfläche des unteren Auflagers lateral ortsunveränderlich gelagert ist, und das zweite Distanzelement des Doppelpaars lateral drehbar um eine fixe Achse, die in der zweiten Teilfläche des oberen Auflagers und in der zweiten Teilfläche des unteren Auflagers lateral ortsunveränderlich gelagert ist.

Ein Doppelpaar von Distanzelementen ist dabei also ein Paar aus einem ersten Distanzelement und einem zweiten Distanzelement, wobei erstes und zweites Distanzelement jeweils ein oberes, zum oberen Auflager gerichtetes Distanzteilelement und ein unteres, zum unteren Auflager gerichtetes Distanzteilelement umfassen, die sich zusammen (d.h. parallel zueinander um dieselben Achslagen) bewegen.

Dabei ist die auf dem Gewinde der Schraubspindel laufende Gewindemutter derart ausgebildet, dass sie Gleitflächen oder eine doppelte Kniehebelstruktur zur ersten und zweiten Teilfläche des oberen Auflagers und zur ersten und zweiten Teilfläche des unteren Auflagers umfasst, die auf Führungsgleitflächen der ersten und zweiten Teilfläche des oberen Auflagers und auf Führungsgleitflächen der ersten und zweiten Teilfläche des unteren Auflagers frei gleitend gelagert sind.

Die Winkelposition zwischen dem ersten Distanzelement und dem zweiten Distanzelement bestimmt dann die laterale Ausdehnung des Platzhalters und ein Abstand der oberen und der unteren Gleitfläche zueinander oder eine Stellung der doppelten Kniehebelstruktur an einer Berührungskante (und damit auch ein Öffnungswinkel der Kniehebelstruktur) und/oder an einer der Position der Gewindemutter zugeordneten Position der oberen und unteren Führungsgleitflächen des oberen und des unteren Auflagers bestimmt die Höhe des Platzhalters.

Weiterhin ist es von besonderem Vorteil, wenn der Platzhalter, der eine Schraubspindel als wesentliches Element des Antriebs der Expansionsvorrichtung enthält, so gestaltet ist, dass diese Schraubspindel auf ihrer zweiten Hälfte ein Gewinde aufweist, das gegenläufig zu einem Gewinde auf der ersten Hälfte der Schraubspindel ist, und die Expansionsvorrichtung ein erstes Distanzelement oder ein erstes Doppelpaar von Distanzelementen, das die erste Hälfte der Schraubspindel nutzt und ein zweites Distanzelement oder ein zweites Doppelpaar von Distanzelementen, das die zweite Hälfte der Schraubspindel nutzt, umfasst. Die jeweilige Wendigkeit des Gewindes der Schraubspindel hängt dabei davon ab, wie das korrespondierende Distanzelement oder Doppelpaar von Distanzelementen relativ zur Achse dieser Schraubspindel gelagert ist.

Dabei sind dem ersten bzw. dem zweiten Distanzelement oder Doppelpaar von Distanzelementen erste bzw. zweite obere und untere dreidimensionale Gleitflächen und entsprechende obere und untere Langlöcher und/oder Führungsgleitflächen zugeordnet. Gegebenenfalls sind zudem jeweils eine oder zwei erste bzw. eine oder zwei zweite bewegliche Achsen sowie eine oder zwei erste bzw. eine oder zwei zweite fixe Achsen dem ersten bzw. dem zweiten Distanzelement oder Doppelpaar von Distanzelementen zugeordnet.

Ein derart ausgestalteter Platzhalter erlaubt damit eine zweiseitige Expansions- und Stützfunktion, was die Stabilität des Platzhalters erhöht und ein "Wegkippen" des Platzhalters verhindert. Dabei müssen die erste Hälfte und die zweite Hälfte der Schraubspindel und der ihr jeweils zugeordneten Elemente nicht symmetrisch zueinander ausgestaltet sein.

Besonders vorteilhaft ist es jedoch, wenn in einem Platzhalter mit einem ersten und einem zweiten Distanzelement oder Doppelpaar von Distanzelementen das erste und das zweite Distanzelement oder Doppelpaar von Distanzelementen zueinander spiegelbildlich arbeitend ausgelegt und angeordnet sind. Der Platzhalter kann dann also eine Symmetrieachse, die senkrecht zur Achse der Schraubspindel verläuft, aufweisen. Ein solches spiegelbildliches Arbeiten ermöglicht eine besonders hohe Stabilität, insbesondere, wenn der Platzhalter als Zwischenwirbelimplantat mittig in den Bandscheibenraum zwischen zwei Wirbelkörper verbracht und dort expandiert wird.

Alternativ ist es jedoch auch möglich, dass in einem Platzhalter mit einem ersten und einem zweiten Distanzelement oder Doppelpaar von Distanzelementen das erste und das zweite Distanzelement oder Doppelpaar von Distanzelementen zueinander gleichläufig ausgelegt und angeordnet ist.

In einem Platzhalter mit einem ersten und einem zweiten Distanzelement kann eine als Antriebselement genutzte Schraubspindel auf Zug belastet sein, um die Distanzelemente während der Expansion zueinander zu führen. Sie kann jedoch auch auf Druck belastet werden, um während der Expansion die Distanzelemente voneinander weg zu führen.

In einer besonders bevorzugten Ausführungsform des erfindungsgemäßen Platzhalters, weist dessen Schraubspindel eine Führungsstruktur zwischen erster und zweiter Hälfte und/oder zwischen erstem und zweitem Distanzelement oder Doppelpaar von Distanzelementen auf, die in einem Rückhalteelement drehbar, aber lateral nicht in ihrer Lage verschiebbar gelagert ist, wobei das Rückhalteelement beweglich, insbesondere vertikal beweglich, aber wiederum lateral nicht in seiner Lage verschiebbar im oberen und im unteren Auflager gelagert ist. Dies dient der Zentrierung der Expansionsvorrichtung in Bezug auf die Auflager.

Der erfindungsgemäße Platzhalter kann in einer Ausführungsform maximal individualisiert werden: Dabei wird die Form und Lage der dreidimensionalen Gleitflächen und die Form und Lage des Langlochs und/oder der Führungsgleitflächen nach einem individuell benötigten Expansionsverhalten gestaltet. Ein solcher Platzhalter kann auf Anforderung nach entsprechend den vom Patienten benötigten Eigenschaften bzw. den vom Chirurgen bevorzugten Verhalten individuell gefertigt werden: Hierzu werden zunächst Untersuchungsdaten des Patienten aufgenommen, die sowohl geometrische Daten des bei dem Patienten bestehenden Problems als auch das Wirbelsäulenmaterial charakterisierende Daten, wie beispielsweise die lokale Knochendichte und -brüchigkeit umfassen. Zudem kann der behandelnde Chirurg weitere Anforderungen hinterlegen, die er für einen bestmöglichen chirurgischen Eingriff sowie für eine optimale Funktion für nötig hält. Aus diesen Daten wird eine optimale Größe des Platzhalters im geschlossenen Zustand und im expandierten Zustand, ggf. eine optimale ineinandergreifende Struktur und ein optimaler Bewegungsverlauf der Veränderung der lateralen Ausdehnung und des Veränderung des vertikalen Abstands von oberer und unterer Auflagefläche in voneinander unabhängiger Art und Weise bestimmt und daraus eine physische Kodierung des Platzhalters, also beispielsweise die Formung von Freiformflächen als Gleitflächen und die Formung von Langlöchern als Gleitlöcher bestimmt. Diese Art der individuellen Bestimmung des für den Patienten und/oder den Chirurgen optimalen Platzhalters kann auch Inhalt einer hierfür vorgesehenen Planungseinheit bzw. eines entsprechenden Computerprogramm-Produktes sein.

Ein für die Implantation mittels minimalinvasivem Eingriff besonders optimierter Platzhalter weist in einer Draufsicht eine nierenförmige (bzw. bohnenförmige) Gestalt auf. Ein solcher Platzhalter weist also lateral höchstens eine Spiegelachse/Symmetrieachse auf, die parallel der Richtung der lateralen Ausdehnung verläuft.

Die nierenförmige bzw. bohnenförmige Gestalt vereinfacht die Implantation in einem bogenförmigen Implantationsweg für eine ventral quer verlaufende Implantation für die transforaminale interkorporelle Fusion.

Für eine schräge, aber geradlinig verlaufende Implantation in das Bandscheibenfach ist eine solche Form jedoch nicht erforderlich.

Bei nur teilweiser chirurgischer Resektion des Wirbelgelenkes (Facettotomie) resultiert ein bogenförmiger Implantationsweg mit einem Implantationstunnel von etwa 8 mm Höhe und 13 mm Breite. Nach biokinemetrischen Analysen sind in der interkorporellen Fusion der Lendenwirbelsäule Implantatshöhen zwischen 7 bis 14 mm erforderlich.

In einer Ausgestaltung weist der erfindungsgemäße Platzhalter eine Minimalhöhe von größer oder gleich 7mm im geschlossenen Zustand und eine Maximalhöhe von kleiner oder gleich 14mm im expandierten Zustand sowie eine laterale Ausdehnung, also eine Ausdehnung parallel zur Richtung des lateralen Auseinanderdriftens, von größer oder gleich 13mm im geschlossenen Zustand auf. Bevorzugt weist er zudem eine laterale Ausdehnung von kleiner oder gleich 25mm im expandierten Zustand auf, aber es sind auch größere laterale Ausdehnungen im expandierten Zustand möglich.

Vorzugsweise steht eine Serie von erfindungsgemäßen Platzhaltern zur Verfügung, die unterschiedliche Höhenbereiche bedienen können. Vorteilhaft ist hier beispielsweise ein kleines Modell des Platzhalters, das eine Minimalhöhe von 7mm und eine Maximalhöhe von 11mm ermöglicht sowie ein großes Modell des Platzhalters, das eine Minimalhöhe von 9mm und eine Maximalhöhe von 14mm ermöglicht. Kleines und großes Modell des Platzhalters sind dann wiederum in nierenförmiger Gestalt oder aber prinzipiell quaderförmiger Gestalt erhältlich. Alle Modelle sind zudem in verschiedenen Einstellungsstufen der Veränderung der lateralen Ausdehnung und des vertikalen Abstands erhältlich.

Zusammenfassend wird also mit dem erfindungsgemäßen Platzhalter der technischen Erfordernis Rechnung getragen, die aus einer biokinemetrischen Betrachtung der intendierten chirurgischen Fusion zweier Lendenwirbelkörper miteinander resultiert, wobei gleichzeitig betrachtet wurden:
- Der mögliche chirurgische Zugangsweg und die resultierenden Platzverhältnisse.
- Die funktionelle Anatomie des zu stabilisierenden Bewegungsabschnittes.
- Die möglichen Bewegungsrichtungen des Implantationsvorgangs innerhalb des chirurgischen Zugangsweges.
- Das nach biokinemetrischen Kriterien optimale Ausmaß des Implantats.

Der erfindungsgemäße Platzhalter in seinen verschiedenen Ausgestaltungen ist bevorzugt in Titan ausgeführt: Titan ist eines der in der Chirurgie aufgrund seiner Beständigkeit und seiner guten Verträglichkeit besonders bevorzugten Materialen, und die hier beschriebene Mechanik des Platzhalters erlaubt eine Ausführung aller seiner Teile in Titan. Jedoch ist auch eine Ausführung in Keramik, oder eine Darstellung mit Polymeren der Gruppe der Polyetherketone (PEK), wie beispielsweise Polyetheretherketon (PEEK) oder in Polyetherketonkoton (PEKK) möglich.

In einem erfindungsgemäßen Verfahren wird der Platzhalter, der als Zwischenwirbelimplantat genutzt wird, nach Entfernung der Bandscheibe (mittels Facettotomie) zwischen eine Wirbelkörpergrundplatte des oberen Wirbelkörpers und eine Wirbelkörperdeckplatte des unteren Wirbelkörpers an Stelle der Bandscheibe minimaliversiv eingeführt, derart, dass das Stellelement der Expansionsvorrichtung des Platzhalters, in einer konkreten Ausführungsform also die Schraubspindel mit ihrem Schraubenkopf mit einem Werkzeug erreichbar bleibt. Ist der Platzhalter in der gewünschten Position, so wird die Schraubspindel mit dem Werkzeug über den Schraubenkopf kontinuierlich gedreht. Dabei expandiert der Platzhalter bevorzugt zunächst in die Breite, um der Wirbelkörpergrundplatte des oberen Wirbelkörpers und der Wirbelkörperdeckplatte des unteren Wirbelkörpers größtmögliches korrespondierendes Auflager zu liefern, um ein Einbrechen oder verzögertes Einsintern zu verhindern. Anschließend wird - zunächst zusätzlich zu einer Fortsetzung der lateralen Expansion - durch fortgesetztes Drehen der Schraubspindel die Expansion des Platzhalters in die Höhe initiiert. Diese vertikale Expansion kann in einer bevorzugten Ausführungsform auch fortgesetzt werden, nachdem die laterale Expansion bereits abgeschlossen ist, also der Platzhalter seine maximale laterale Ausdehnung erreicht hat - wiederum durch fortgesetztes Drehen der Schraubspindel in immer dieselbe Richtung, bis die Maximalhöhe des Platzhalters erreicht ist. Die vertikale Expansion kann im Extremfall auch erst beginnen, wenn die laterale Expansion abgeschlossen ist. Hiernach wird das Werkzeug aus der Wundöffnung entfernt, und der Platzhalter verbleibt fest auf seinem Platz (anstelle der Bandscheibe) zwischen der Wirbelkörpergrundplatte des oberen Wirbelkörpers und der Wirbelkörperdeckplatte des unteren Wirbelkörpers, die jetzt im richtigen Abstand zueinander durch den expandierten Platzhalter gestützt werden.

Es gibt nun verschiedene Möglichkeiten, die Lehre der vorliegenden Erfindung in vorteilhafter Weise auszugestalten und weiterzubilden und/oder die oben beschriebenen Ausführungsformen - soweit möglich - miteinander zu kombinieren. Dazu ist einerseits auf die dem Patentanspruch 1 nachgeordneten Patentansprüche und andererseits auf die nachfolgende Erläuterung der Ausführungsbeispiele der Erfindung anhand der Zeichnung zu verweisen.

Es zeigen:
- die Fig. 1a - 1d ein erstes Ausführungsbeispiel eines erfindungsgemäßen Platzhalters für die Wirbelsäulenchirurgie im geschlossenen Zustand und im expandierten, also geöffneten und angehobenen Zustand;
- die Fig. 2a und 2b ein zweites Ausführungsbeispiel eines erfindungsgemäßen Platzhalters für die Wirbelsäulenchirurgie im geschlossenen Zustand und im expandierten Zustand;
- die Fig. 3a - 3c ein drittes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand;

- die Fig. 4 ein viertes Ausführungsbeispiel eines erfindungsgemäßen Platzhalters für die Wirbelsäulenchirurgie im geschlossenen Zustand;
- die Fig. 5a - 5c eine Innenansicht des vierten Ausführungsbeispiels eines erfindungsgemäßen Platzhalters im geschlossenen Zustand, im geöffneten Zustand und im expandierten, also geöffneten und angehobenen Zustand;
- die Fig. 6a - 6c ein fünftes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand;
- die Fig. 7a - 7c eine Innenansicht des fünften Ausführungsbeispiels eines erfindungsgemäßen Platzhalters im geschlossenen Zustand, im geöffneten Zustand und im expandierten, also geöffneten und angehobenen Zustand;
- die Fig. 8a - 8c ein sechstes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand;
- die Fig. 9a - 9c eine Draufsicht des sechsten Ausführungsbeispiels eines erfindungsgemäßen Platzhalters im geschlossenen Zustand, im geöffneten Zustand und im expandierten, also geöffneten und angehobenen Zustand;
- die Fig. 10 eine Explosionsdarstellung des sechsten Ausführungsbeispiels eines erfindungsgemäßen Platzhalters;
- die Fig. 11a - 11c eine Innenansicht des sechsten Ausführungsbeispiels eines erfindungsgemäßen Platzhalters im geschlossenen Zustand, im geöffneten Zustand und im expandierten, also geöffneten und angehobenen Zustand;
- die Fig. 12a - 12c ein siebtes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand;
- die Fig. 13a - 13d eine Innenansicht des siebten Ausführungsbeispiels eines erfindungsgemäßen Platzhalters im geschlossenen Zustand, im geöffneten Zustand, im geöffneten und teilweise angehobenen Zustand sowie im expandierten, also vollständig geöffneten und angehobenen Zustand;
- die Fig. 14 eine Draufsicht des siebten Ausführungsbeispiels eines erfindungsgemäßen Platzhalters;
- die Fig. 15a und 15b eine perspektivische Ansicht des siebten Ausführungsbeispiels eines erfindungsgemäßen Platzhalters;
- die Fig. 16a - 16c ein achtes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand in einer perspektivischen Ansicht;
- die Fig. 17a - 17c eine Innenansicht des achten Ausführungsbeispiels eines erfindungsgemäßen Platzhalters im geschlossenen Zustand, im geöffneten Zustand, sowie im expandierten, also vollständig geöffneten und angehobenen Zustand in einer perspektivischen Ansicht;
- die Fig. 18a - 18d ein neuntes Ausführungsbeispiel eines erfindungsgemäßen Platzhalters für die Wirbelsäulenchirurgie im geschlossenen Zustand, im geöffneten Zustand, im expandierten, also geöffneten und angehobenen Zustand, sowie im expandierten und arretierten Zustand in einer perspektivischen Ansicht;
- die Fig. 19a - 19d eine Innenansicht des neunten Ausführungsbeispiels eines erfindungsgemäßen Platzhalters im geschlossenen Zustand, im geöffneten Zustand, im expandierten, also geöffneten und angehobenen Zustand, sowie im expandierten und arretierten Zustand in einer Draufsicht;
- die Fig. 20a - 20d eine Innenansicht des neunten Ausführungsbeispiels eines erfindungsgemäßen Platzhalters im geschlossenen Zustand, im geöffneten Zustand, im expandierten, also geöffneten und angehobenen Zustand, sowie im expandierten und arretierten Zustand in einer perspektivischen Ansicht;
- die Fig. 21a - 21c ein zehntes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand in einer Seitenansicht und einer Draufsicht;
- die Fig. 22a - 22c eine Innenansicht des zehnten Ausführungsbeispiels eines erfindungsgemäßen Platzhalters im geschlossenen Zustand, im geöffneten Zustand und im expandierten, also geöffneten und angehobenen Zustand in einer perspektivischen Ansicht;
- die Fig. 23a - 23c eine Innenansicht des zehnten Ausführungsbeispiels eines erfindungsgemäßen Platzhalters im geschlossenen Zustand, im geöffneten Zustand und im expandierten, also geöffneten und angehobenen Zustand in einer Draufsicht und einer Seitenansicht;
- die Fig. 24a - 24c eine weitere Innenansicht des zehnten Ausführungsbeispiels eines erfindungsgemäßen Platzhalters im geschlossenen Zustand, im geöffneten Zustand und im expandierten, also geöffneten und angehobenen Zustand in einer perspektivischen Ansicht:
- die Fig. 25a - 25c ein elftes Ausführungsbeispiel eines erfindungsgemäßen Platzhalters für die Wirbelsäulenchirurgie im geschlossenen Zustand, im geöffneten Zustand und im expandierten, also geöffneten und angehobenen Zustand in einer perspektivischen Ansicht;
- die Fig. 26a - 26d eine Innenansicht des elften Ausführungsbeispiels eines erfindungsgemäßen Platzhalters im geschlossenen Zustand, im geöffneten Zustand, im expandierten, also geöffneten und angehobenen Zustand, sowie im expandierten und arretierten Zustand in einer perspektivischen Ansicht;
- die Fig. 27a und 27b zwei Varianten der Implantation des Platzhalters in einer Funktion als Zwischenwirbelkörperimplantat.

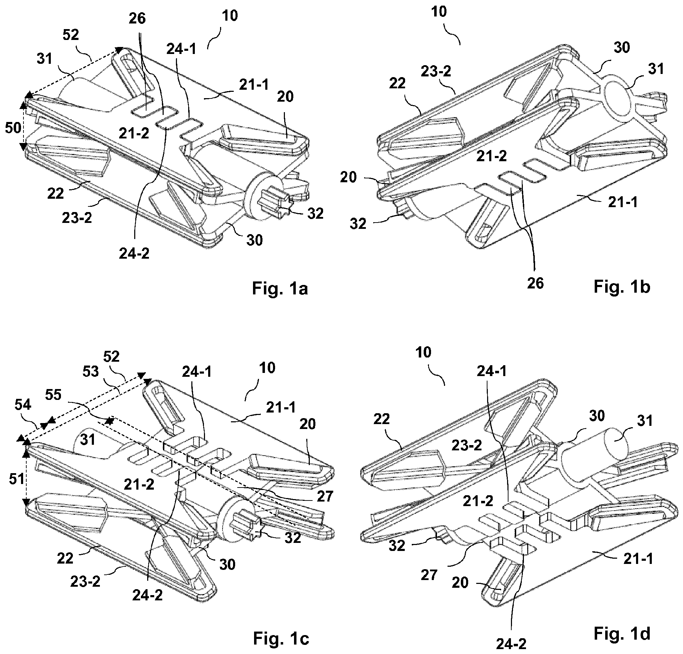

Die Fig. 1a - 1d zeigen ein erstes Ausführungsbeispiel eines erfindungsgemäßen Platzhalters 10 für die Wirbelsäulenchirurgie in jeweils zwei perspektivischen Ansichten im geschlossenen Zustand und im expandierten, also geöffneten und angehobenen Zustand, der ein oberes Auflager 20 mit einer oberen Auflagefläche 21 und ein unteres Auflager 22 mit einer unteren Auflagefläche 23 umfasst, deren relative Lage zueinander veränderbar ist, wobei die obere 21 wie auch die untere Auflagefläche 23 jeweils eine erste 21-1, 23-1 und ein zweite Teilfläche 21-2, 23-2 aufweist, die sich in einem geschlossenen Zustand des Platzhalters 10 an einer Kante 24-1, 24-2, 25-1, 25-1 berühren. Der Platzhalter umfasst zudem eine Expansionsvorrichtung 30, mit der die Auflageflächen 21, 23 in ihrer lateralen Ausdehnung durch laterales Auseinanderdriften der ersten 21-1, 23-1 und zweiten Teilfläche 21-2, 23-2 bis zu einem Maximaldriftbetrag 54 zwischen einer minimalen lateralen Ausdehnung 52 und einer maximalen lateralen Ausdehnung 53 wie auch in ihrem vertikalen Abstand zwischen einer Minimalhöhe 50 und einer Maximalhöhe 51 des Platzhalters 10 zueinander veränderbar sind, so dass der Platzhalter 10 zwischen einem geschlossenen und einem expandiertem Zustand einstellbar ist.

Um eine Expansion in einfachster Art und Weise nach dem Verbringen des Platzhalters 10 an seinen Wirkungsort in der Wirbelsäule des Patienten zu realisieren, enthält die Expansionsvorrichtung 30 einen einzigen Antrieb, d.h., die Durchführung der Expansion, und damit sowohl der Veränderung der lateralen Ausdehnung der Auflageflächen 21, 23 als auch die Veränderung des vertikalen Abstands wird über ein und denselben Antrieb realisiert. Dieser Antrieb weist als wesentliches Element eine Schraubspindel 31 auf, mittels der die Expansion des Platzhalters 10 ausgeführt werden kann. Die Schraubspindel 31 ist in diesem ersten Ausführungsbeispiel eines erfindungsgemäßen Platzhalters 10 Teil eines Keilmechanismus bzw. präziser eines dem Prinzip von Keil und Gegenkeil beruhendem Mechanismus, mit dem die Veränderung der lateralen Ausdehnung wie auch des vertikalen Abstands der Auflageflächen 21, 23 gleichzeitig und in voneinander abhängiger Weise - im gleichen Verhältnis zueinander - betrieben wird.

Zwei gedachte gerade Pyramiden stehen einander im definierten Abstand Spitze an Spitze spiegelbildlich in der Weise gegenüber, dass ihre beiden Höhen auf einer gemeinsamen Geraden liegen. Jeweils zwei einander gegenüberliegende, materiell ausgebildete Seitenkanten bilden die Keile, die einen gemeinsamen Gegenkeil (∇ Nabla) in seiner räumlichen Position ändern, wenn die Pyramiden sich annähern.

Der Platzhalter kann als Wirbelsäulenimplantat im Sinne eines Abstandhalters bzw. Zwischenwirbelimplantats eine Bandscheibe oder im Sinne eines Wirbelkörperersatzes einen Wirbelkörper ersetzen, oder auch im Sinne einer Augmentierung eines Wirbelkörpers im Wirbelkörper selbst zur Anwendung kommen (Spondyloplastie).

Die erste 21-1, 23-1 wie auch die zweite Teilfläche 21-2, 23-2 der oberen 21 wie auch der unteren Auflagefläche 23 weisen an der Kante 24-1, 24-2, 25-1, 25-1, an der sie sich im geschlossenen Zustand berühren, eine ineinandergreifende Struktur 26 in Form von ineinandergreifenden Zähnen auf. Diese Struktur ist also derart gestaltet, dass sie bei einer Expansion ein laterales Auseinanderdriften der ersten 21-1, 23-1 und zweiten Teilfläche 21-2, 23-2 ermöglicht, und im expandierten Zustand ein senkrecht zur Richtung des lateralen Auseinanderdriftens durch die oberen 21 wie auch die untere Auflagefläche 23 verlaufender lateraler Spalt 27 eine Spaltbreite 55 aufweist, die kleiner ist als der Maximaldriftbetrag 54.

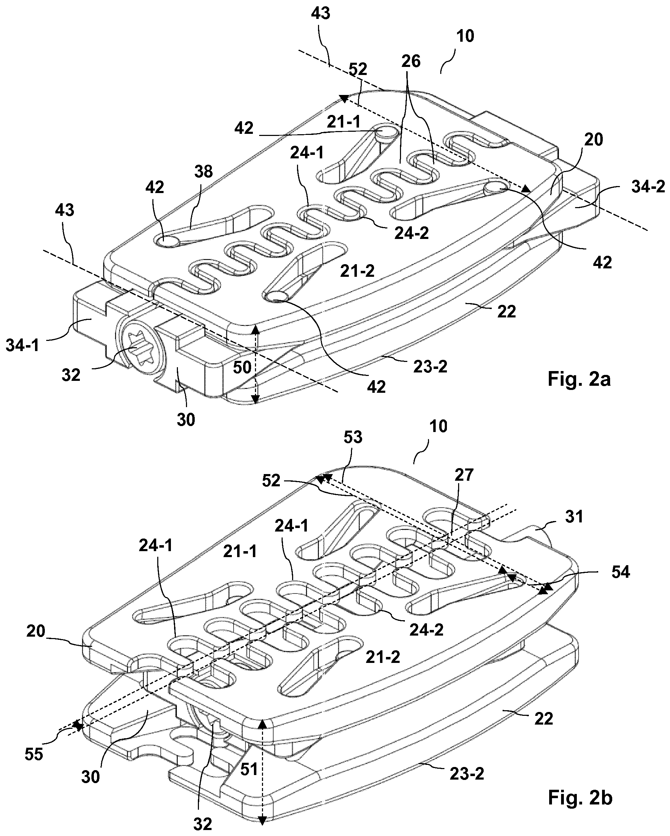

In den Fig. 2a und 2b wird ein zweites Ausführungsbeispiel eines erfindungsgemäßen Platzhalters 10 für die Wirbelsäulenchirurgie in einer perspektivischen Ansicht im geschlossenen Zustand und im expandierten Zustand dargestellt. Dieser Platzhalter 10 umfasst wiederum ein oberes Auflager 20 mit einer oberen Auflagefläche 21 und ein unteres Auflager 22 mit einer unteren Auflagefläche 23, deren relative Lage zueinander veränderbar ist, wobei die obere 21 wie auch die untere Auflagefläche 23 jeweils eine erste 21-1, 23-1 und ein zweite Teilfläche 21-2, 23-2 aufweist, die sich in einem geschlossenen Zustand des Platzhalters 10 an einer Kante 24-1, 24-2, 25-1, 25-1 berühren. Der Platzhalter umfasst zudem eine Expansionsvorrichtung 30, mit der die Auflageflächen 21, 23 in ihrer lateralen Ausdehnung durch laterales Auseinanderdriften der ersten 21-1, 23-1 und zweiten Teilfläche 21-2, 23-2 bis zu einem Maximaldriftbetrag 54 zwischen einer minimalen lateralen Ausdehnung 52 und einer maximalen lateralen Ausdehnung 53 wie auch in ihrem vertikalen Abstand zwischen einer Minimalhöhe 50 und einer Maximalhöhe 51 des Platzhalters 10 zueinander veränderbar sind, so dass der Platzhalter 10 zwischen einem geschlossenen und einem expandiertem Zustand einstellbar ist.

In diesem zweiten Ausführungsbeispiel des erfindungsgemäßen Platzhalters 10 weisen die erste 21-1, 23-1 wie auch die zweite Teilfläche 21-2, 23-2 der oberen 21 wie auch der unteren Auflagefläche 23 an der Kante 24-1, 24-2, 25-1, 25-1, an der sie sich im geschlossenen Zustand berühren, eine wellenförmige ineinandergreifende Struktur 26 auf. Auch diese Struktur ist also derart gestaltet, dass sie bei einer Expansion ein laterales Auseinanderdriften der ersten 21-1, 23-1 und zweiten Teilfläche 21-2, 23-2 ermöglicht, und im expandierten Zustand ein senkrecht zur Richtung des lateralen Auseinanderdriftens durch die oberen 21 wie auch die untere Auflagefläche 23 verlaufender lateraler Spalt 27 eine Spaltbreite 55 aufweist, die wesentlich kleiner ist als der Maximaldriftbetrag 54. Im geschlossenen Zustand des Platzhalters 10 zeigen die ineinandergreifenden Strukturen der ersten und zweiten Teilfläche der oberen wie auch der unteren Auflageflächen ein Ineinandergreifen nach dem "Schlüssel-Schloß-Prinzip" über die gesamte Länge des Platzhalters, so dass im geschlossenen Zustand die obere wie auch die untere Auflagefläche als jeweils eine durchgehende Fläche wahrgenommen werden kann.

Die Expansionsvorrichtung 30 dieses zweiten Ausführungsbeispiels des erfindungsgemäßen Platzhalters 10 ist nun zudem eingerichtet, die Veränderung der lateralen Ausdehnung und des vertikalen Abstands in zwei voneinander unabhängig und frei definierbaren Bewegungsverläufen aber mittels eines einzigen Antriebs auszuführen. Hierzu umfasst die Expansionsvorrichtung des Platzhalters 10 ein bewegliches Distanzelement 34, das entlang der Schraubspindel 31 durch Drehung des Schraubenkopfes 32 bewegbar ist und vier Führungselemente 42 in Form von Pilzkopfbolzen aufweist, die jeweils in einem ersten oberen Langloch 38 in der ersten Teilfläche 21-1 und in einem zweiten oberen Langloch 38 der zweiten Teilfläche 21-2 des oberen Auflagers 20 und in einem ersten unteren Langloch 39 der ersten Teilfläche 23-1 und in einem zweiten unteren Langloch 39 der zweiten Teilfläche 23-2 des unteren Auflagers 22 verschiebbar sind.

Das Distanzelement 34 selbst weist obere und untere dreidimensionale Gleitflächen 40, 41 auf, die auf dem Distanzelement 34 so zueinander geformt sind, und die Langlöcher 38 in jeder der Teilflächen des oberen und unteren Auflagers so geformt und angeordnet sind, dass für eine definierte Position, die das Distanzelement 34 auf der Schraubspindel 31 einnimmt, die dazu korrespondierende Position der Führungselemente 42 in Form vom Pilzkopfbolzen in den entsprechend geformten Langlöchern 38, 39 die laterale Ausdehnung des Platzhalters 10 und deren Abstand der oberen 40 und der unteren dreidimensionalen Gleitfläche 41 zueinander auf dem Distanzelement 34 jeweils an einer Berührungskante 43 des oberen 20 und des unteren Auflagers 22 die Höhe des Platzhalters 10 bestimmt. Da in diesem Ausführungsbespiel auch die Innenseite des oberen und unteren Auflagers 20, 22 eine Formung aufweist, "wandert" dabei die Berührungskante 43 während der Expansion des Platzhalters.

Die Expansionsvorrichtung 30 umfasst ein erstes Distanzelement 34-1, das die erste Hälfte der Schraubspindel nutzt und ein zweites Distanzelement 34-2, das die zweite Hälfte der Schraubspindel 31 nutzt, wobei erstem 34-1 und zweitem Distanzelement 34-2 erste bzw. zweite obere und untere dreidimensionale Gleitflächen und entsprechende obere 38 und untere Langlöcher 39 zugeordnet sind.

Dieses zweite Ausführungsbeispiel des erfindungsgemäßen Platzhalters 10 arbeitet deshalb zwar mit einem Keil- und Gegenkeil-Prinzip, jedoch wird durch ein Gleiten der an den Innenseiten geformten Auflager auf den entsprechend des gewünschten vertikalen Bewegungsablaufs frei geformten Gleitflächen der Keile und der darauf angeordneten Pilzköpfe, die als Führungselemente 42 in entsprechend des gewünschten lateralen Bewegungsablaufs frei geformten Langlöchern 38, 39 in den Teilflächen 21-1, 21-2, 23-1, 23-2 des oberen und unteren Auflagers 20, 22 gleiten können, das bislang feste Verhältnis der lateralen zur vertikalen Expansion bei Nutzung eines Keil- und Gegenkeil-Prinzips durchbrochen, so dass die Keile als entsprechende Distanzelemente 34-1, 34-2 agieren.

Die Teilflächen der Auflager können innerhalb der Nablas und Deltas (aufgrund deren räumlicher Ausdehnung bzw. Formung) auf Bahnen laufen, die die Expansion in Breite und Höhe nachvollziehen. Selbst wenn die Keile und die Innenseiten der Auflager ebene Gleitflächen aufweisen und damit eine lineare Änderung des vertikalen Abstands erreicht wird, so ist der Ablaufplan der lateralen Ausdehnung davon unabhängig in der Konfiguration des Langloches niedergelegt. Diese kann damit nichtlinear derart erfolgen, dass zunächst eine schnelle laterale Ausdehnung einsetzt, während beim Erreichen eines gewissen vertikalen Abstands (und - bei Einsatz als Zwischenwirbelimplantat - auch des Aufbaus eines gewissen Drucks auf den Platzhalter durch den oberen und unteren Wirbelkörper) nur eine nur noch geringe laterale Ausdehnung erfolgt und schließlich der letzte Bereich der Änderung des vertikalen Abstands ohne weitere Änderung der lateralen Ausdehnung erfolgen kann.

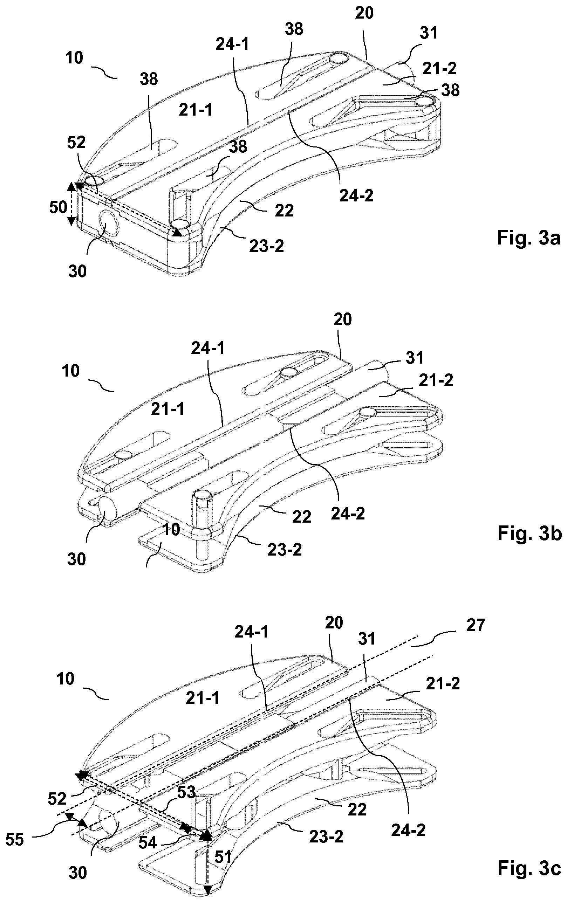

Die Fig. 3a - 3c zeigt ein drittes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand jeweils in perspektivischen Ansichten.

Dieses dritte Ausführungsbeispiel des erfindungsgemäßen Platzhalters 10 funktioniert im Wesentlichen wie das zweite Ausführungsbeispiel, und wird damit auch an den entsprechenden Stellen durch das zweite Ausführungsbeispiel beschrieben. Er weist jedoch drei Besonderheiten auf, in denen er sich vom zweiten Ausführungsbeispiel unterscheidet:
- Der Platzhalter 10 weist keine ineinandergreifende Struktur an der Kante 24-1, 24-2, 25-1, 25-1, an der sich die erste 21-1, 23-1 und die zweite Teilfläche 21-2, 23-2 der oberen 21 und der unteren Auflagefläche 23 berühren, auf. Damit entsteht im Verlaufe der Expansion ein lateraler Spalt 27, der hier auch als mittlerer, medianer bzw. intermediärer Spalt bezeichnet werden kann, dessen Breite 55 dem Maximaldriftbetrag 54 der lateralen Ausdehnung der oberen 21 und unteren Auflageflächen 23 entspricht.
- Die Langlöcher 38, 39, in denen die als Führungselemente genutzten Pilzköpfe, die auf den keilförmigen Distanzelementen angeordnet sind, gleiten, weisen eine Formung derart auf, dass zunächst eine lineare Veränderung der lateralen Ausdehnung bis zu einem Maximaldriftbetrag 54 erfolgt, während der eine lineare Veränderung der der lateralen Position der Teilflächen der jeweiligen Auflageflächen und damit der lateralen Ausdehnung des Platzhalters einsetzt, dann aber weiterhin eine lineare Veränderung des vertikalen Abstands bzw. des vertikalen Hubs erfolgt, während die laterale Ausdehnung konstant bleibt: Sowohl die Veränderung der lateralen Ausdehnung als auch die Veränderung des vertikalen Abstands erfolgen linear, aber versetzt zueinander. Der Algorithmus dieser Abfolge ist in der Ausformung der Langlöcher kodiert.
- Der Platzhalter 10 weist eine nierenförmige Gestalt auf, ist also insbesondere für eine Implantation auf einem bogenförmigen Implantationsweg geeignet.

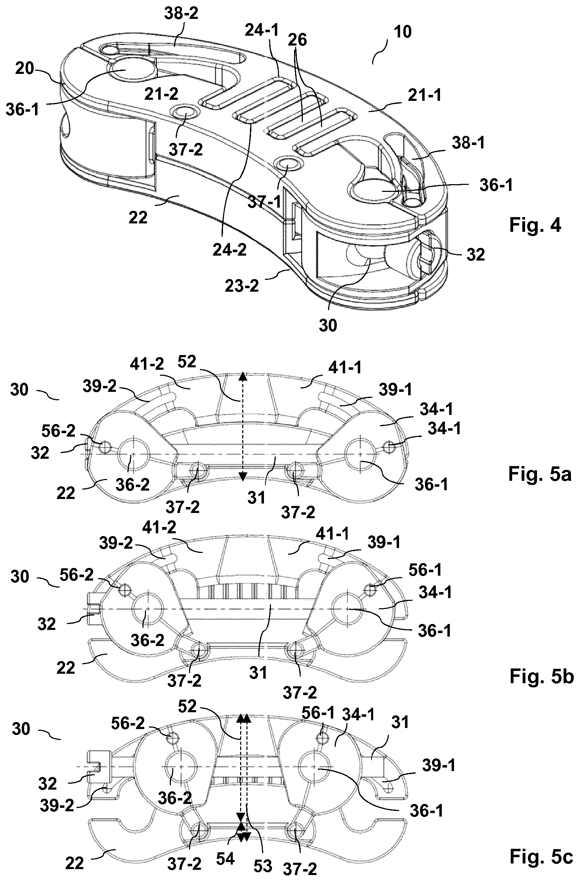

In der Fig. 4 ist ein viertes Ausführungsbeispiel eines erfindungsgemäßen Platzhalters 10 für die Wirbelsäulenchirurgie im geschlossenen Zustand dargestellt, von dem die Fig. 5a - 5c eine Innenansicht im geschlossenen Zustand, im geöffneten Zustand und im expandierten, also geöffneten und angehobenen Zustand zeigen.

Auch das vierte Ausführungsbeispiel des erfindungsgemäßen Platzhalters für die Wirbelsäulenchirurgie umfasst ein oberes Auflager 20 mit einer oberen Auflagefläche 21 und ein unteres Auflager 22 mit einer unteren Auflagefläche 23, deren relative Lage zueinander veränderbar ist, wobei die obere 21 wie auch die untere Auflagefläche 23 jeweils eine erste 21-1, 23-1 und ein zweite Teilfläche 21-2, 23-2 aufweist, die sich in einem geschlossenen Zustand des Platzhalters 10 an einer Kante 24-1, 24-2, 25-1, 25-1 berühren. Der Platzhalter umfasst zudem eine Expansionsvorrichtung 30, mit der die Auflageflächen 21, 23 in ihrer lateralen Ausdehnung durch laterales Auseinanderdriften der ersten 21-1, 23-1 und zweiten Teilfläche 21-2, 23-2 der Auflageflächen 21, 23 bis zu einem Maximaldriftbetrag 54 zwischen einer minimalen lateralen Ausdehnung 52 und einer maximalen lateralen Ausdehnung 53 wie auch in ihrem vertikalen Abstand zwischen einer Minimalhöhe 50 und einer Maximalhöhe 51 des Platzhalters 10 zueinander veränderbar sind, so dass der Platzhalter 10 zwischen einem geschlossenen und einem expandiertem Zustand einstellbar ist.

Und auch in diesem vierten Ausführungsbeispiel des erfindungsgemäßen Platzhalters 10 weisen die erste 21-1, 23-1 wie auch die zweite Teilfläche 21-2, 23-2 der oberen 21 wie auch der unteren Auflagefläche 23 an der Kante 24-1, 24-2, 25-1, 25-1, an der sie sich im geschlossenen Zustand berühren, eine ineinandergreifende Struktur 26 in Form von ineinandergreifenden Zähnen auf. Diese Struktur ist derart gestaltet, dass sie bei einer Expansion ein laterales Auseinanderdriften der ersten 21-1, 23-1 und zweiten Teilfläche 21-2, 23-2 ermöglicht, und im expandierten Zustand ein senkrecht zur Richtung des lateralen Auseinanderdriftens durch die oberen 21 wie auch die untere Auflagefläche 23 verlaufender lateraler Spalt eine Spaltbreite aufweist, die kleiner ist als der Maximaldriftbetrag: In diesem Fall ist die Spaltbreite im zentralen Bereich der Auflageflächen 21, 23 Null, nur in den Randbereichen der Auflageflächen tritt noch ein echter lateraler Spalt auf.

Die Expansionsvorrichtung 30 des vierten Ausführungsbeispiels des erfindungsgemäßen Platzhalters 10 umfasst des Weiteren ein bewegliches Distanzelement 34, das sich von den bisher beschriebenen Distanzelementen unterscheidet: Es ist an einer Position auf dem Distanzelement 34 lateral drehbar gelagert ist um eine bewegliche Achse 36, die mittels einer in die bewegliche Achse 36 integrierte und auf einem Gewinde 33 der Schraubspindel 31 laufenden Gewindemutter entlang der Schraubspindel 31 verschiebbar ist, wobei die bewegliche Achse 36 indirekt in einem oberen Langloch 38 in der ersten Teilfläche 21-1 des oberen Auflagers 20 und in einem unterem Langloch 39 in der ersten Teilfläche 23-1 des unteren Auflagers 22 laufend angeordnet ist. Das Distanzelement ist des Weiteren lateral drehbar ist um eine fixe Achse 37, die in der zweiten Teilfläche 21-2 des oberen Auflagers 20 und in der zweiten Teilfläche 23-2 des unteren Auflagers 22 lateral ortsunveränderlich gelagert ist.

Zudem ist das Distanzelement an einem ersten und/oder einem zweiten Ende frei gleitend auf einer oberen dreidimensionalen Gleitfläche 40 unter der ersten 21-1 und/oder der zweiten Teilfläche 21-2 des oberen Auflagers 20 sowie auf einer unteren dreidimensionalen Gleitfläche 41 über der ersten 23-1 und/oder zweiten Teilfläche 23-2 des unteren Auflagers 22 gelagert, wobei die obere dreidimensionale Gleitfläche 40 und die untere dreidimensionale Gleitfläche 41 so zueinander geformt sind, dass das erste und/oder zweite Ende des Distanzelements 34 für jeden Drehwinkel des Distanzelements 34 um die fixe Achse 37 eine definierte Position auf der oberen 40 wie auch auf der unteren dreidimensionalen Gleitfläche 41 einnimmt, deren absolute Lage und die dazu korrespondierende Position der beweglichen Achse 36 in den entsprechend geformten Langlöchern 38, 39 die laterale Ausdehnung des Platzhalters 10 und deren Abstand der oberen 40 und der unteren dreidimensionalen Gleitfläche 41 zueinander im geschlossenen Zustand die Höhe des Platzhalters 10 bestimmt.

Dabei umfasst in diesem vierten Ausführungsbeispiel des erfindungsgemäßen Platzhalters 10 die Expansionsvorrichtung 30 ein erstes Distanzelement 34-1, das die erste Hälfte 31-1 der Schraubspindel nutzt und ein zweites Distanzelement 34-2, das die zweite Hälfte 31-2 der Schraubspindel 31 nutzt. Erstem 34-1 und zweitem Distanzelement 34-2 sind erste 40-1, 41-1 bzw. zweite obere und untere dreidimensionale Gleitflächen 40-2, 41-2, jeweils erste bzw. zweite bewegliche Achse 36-1, 36-2 sowie erste bzw. zweite fixe Achse 37-1, 37-2 und entsprechende obere 38-1, 38-2 und untere Langlöcher 39-1, 39-2 zugeordnet.

Das vierte Ausführungsbeispiel des erfindungsgemäßen Platzhalters 10 weist wiederum eine nierenförmige Gestalt auf, ist also insbesondere für eine Implantation auf einem bogenförmigen Implantationsweg geeignet. Nach seiner Verbringung an seinen Wirkungsort kann dieser Platzhalter dann mit den Distanzelementen spiegelbildlich arbeitend expandiert werden, was sehr vorteilhaft ist, wenn er beispielsweise in eine zentrale Lage anstelle der Bandscheibe zwischen zwei Wirbelkörper verbracht wurde und bei der Expansion ein gleichmäßiger Druck über den gesamten zentralen Bereich ausgeübt werden soll.

Die Lage und Form der Langlöcher und der dreidimensionalen Gleitflächen des vierten Ausführungsbeispiels sind derart gestaltet, dass eine nichtlineare laterale Expansion und eine lineare vertikale Expansion erfolgt.

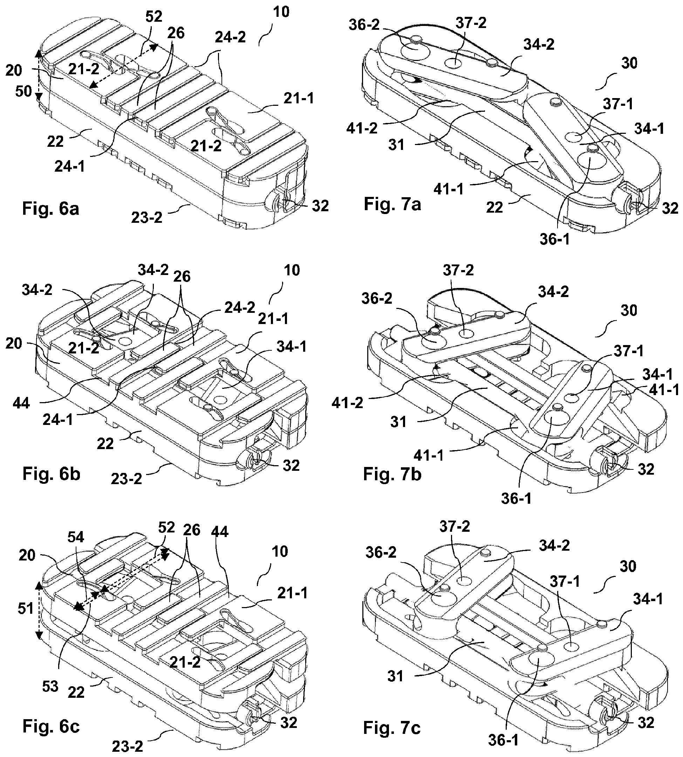

Die Fig. 6a - 6c zeigen ein fünftes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand - wiederum in einer perspektivischen Ansicht, während in den Fig. 7a - 7c eine Innenansicht des fünften Ausführungsbeispiels eines erfindungsgemäßen Platzhalters 10 im geschlossenen Zustand, im geöffneten Zustand und im expandierten, also geöffneten und angehobenem Zustand dargestellt ist- in derselben perspektivischen Ansicht.

Für dieses fünfte Ausführungsbeispiel des erfindungsgemäßen Platzhalters 10 gilt das für das vierte Ausführungsbeispiel gesagte, jedoch unterscheidet es sich von diesem vierten Ausführungsbeispiel in den folgenden Besonderheiten:
- Das Distanzelement 34 bzw. die Distanzelemente 34-1, 34-2 sind mittels Nocken realisiert, wobei die Enden der Nocken auf dreidimensionalen Gleitflächen 41-1, 41-2 gleitend gelagert sind.
- Die erste 21-1, 23-1 wie auch die zweite Teilfläche 21-2, 23-2 der oberen 21 wie auch der unteren Auflagefläche 23 weisen an der Kante 24-1, 24-2, 25-1, 25-1, an der sie sich im geschlossenen Zustand berühren, eine ineinandergreifende Struktur 26 in Form von ineinandergreifenden Zähnen auf. Diese Struktur setzt sich - teilweise periodisch, teilweise in unregelmäßigen Abständen über die gesamte Länge der oberen 21 wie auch der unteren Auflagefläche 23 fort und ist derart gestaltet, dass sie bei einer Expansion ein laterales Auseinanderdriften der jeweils ersten 21-1, 23-1 und zweiten Teilfläche 21-2, 23-2 ermöglicht. Die Struktur der ineinandergreifenden Zähne verläuft jedoch über die gesamte laterale Breite des Platzhalters 10 im geschlossenen Zustand, so dass auch im expandierten Zustand kein lateraler Spalt entsteht, also die Spaltbreite eines gedachten lateralen Spalts senkrecht zur Richtung des lateralen Auseinanderdriftens gleich Null ist. Mehr noch: Das obere 20 wie auch das untere Auflager 22 ist derart geformt, dass die ineinandergreifende Zahnstruktur 26 der ersten Teilfläche 21-1, 23-1 auf einer Auflagestruktur 44 im zur zweiten Teilfläche 21-2, 23-2 zugehörigen Teil des Auflagers 20, 22 und die ineinandergreifende Zahnstruktur 26 der zweiten Teilfläche 21-2, 23-2 auf einer Auflagestruktur 44 im zur ersten Teilfläche 21-1, 23-1 zugehörigen Teil des Auflagers 20, 22 beim lateralen Auseinanderdriften während der Expansion des Platzhalters 10 noch stabiler und sicherer aneinander gleiten kann.

- Das fünfte Ausführungsbeispiel des erfindungsgemäßen Platzhalters 10 weist in seiner Draufsicht eine im weitesten Sinne quaderförmige Grundform auf, wenn auch mit abgerundeten Ecken. Es eignet sich deshalb besonders für einen geraden Implantationsweg 70, jedoch ist seine Verwendung aufgrund seiner abgerundeten Ecken auch auf einem bogenförmigen Implantationsweg 70 nicht ausgeschlossen.
- Die als zentrales Antriebselement genutzte Schraubspindel 31 ist in jeglichem Zustand des Platzhalters 10 in einen Randbereich verbracht, was eine Betätigung der Schraubspindel 31 mittels einen in den Schraubenkopf 32 greifenden Werkzeugs bei entsprechender Platzierung des Platzhalters 10 an seinem Wirkungsort ventral vor dem Wirbelkanal der Wirbelsäule erleichtert.
- Betrachtet man das Innenleben des fünften Ausführungsbeispiels des erfindungsgemäßen Platzhalters 10 genauer, so ist ersichtlich, dass die Stellung der Nocken 34-1, 34-2 zur Achse der Schraubspindel 31 im geschlossenen Zustand des Platzhalters 10 ca. 20°, im zunächst nur lateral geöffneten Zustand des Platzhalters ca. 70° und im expandierten, d.h. geöffneten und angehobenen Zustand ca. 100° beträgt. Die Veränderung des vertikalen Abstands und damit der vertikale Hub erfolgt also relativ schnell am Ende der Expansion des Platzhalters bei Gleiten der Nocken 34-1, 34-2 über einen kurzen aber steil ansteigendem Bereich der dreidimensionalen Gleitflächen 40-1, 40-2, 41-1, 41-2.

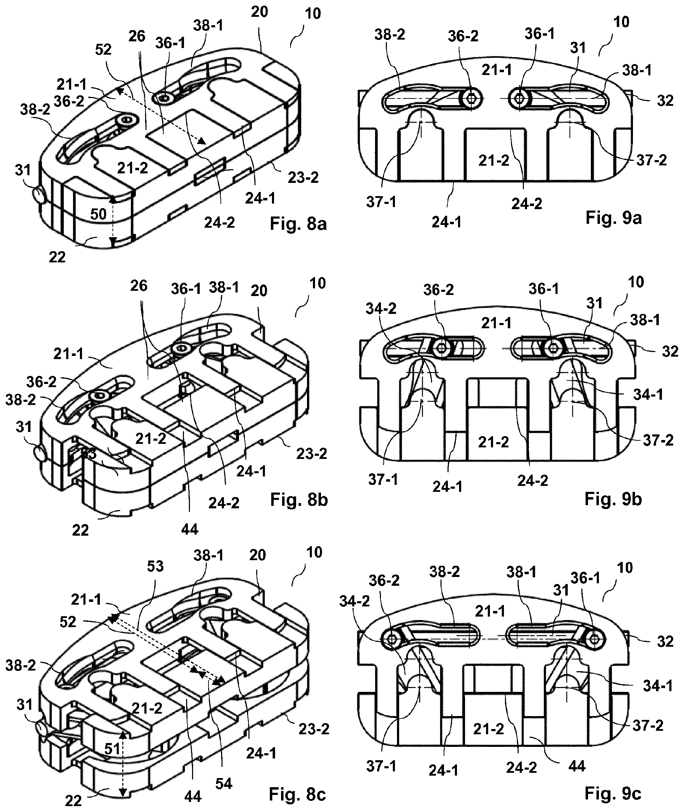

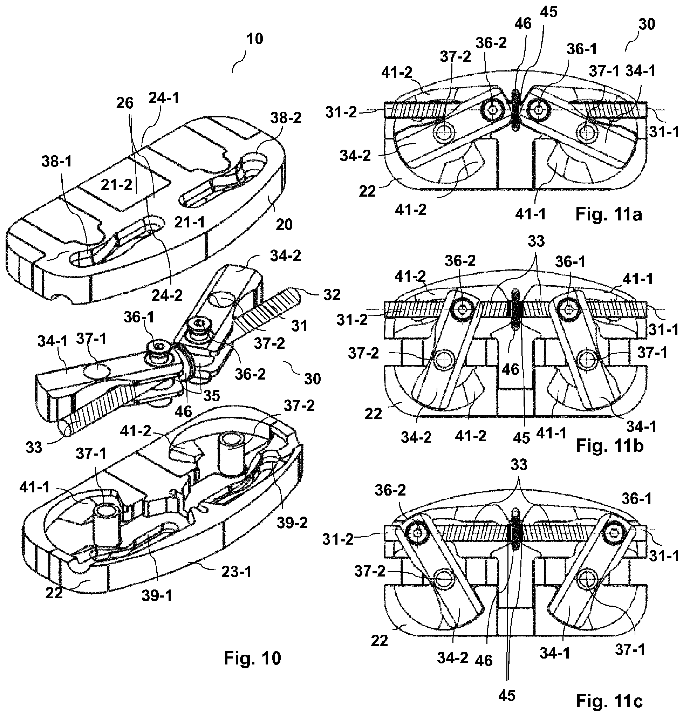

In den Fig. 8a - 8c ist ein sechstes Ausführungsbeispiel eines erfindungsgemäßen Platzhalters 10 für die Wirbelsäulenchirurgie im geschlossenen Zustand, im geöffneten Zustand und im expandierten, also geöffneten und angehobenem Zustand jeweils in einer perspektivischen Ansicht, in den Fig. 9a - 9c eine Draufsicht auf das sechste Ausführungsbeispiel eines erfindungsgemäßen Platzhalters 10 im geschlossenen Zustand, im geöffneten Zustand und im expandierten, also geöffneten und angehobenem Zustand; in der Fig. 10 eine Explosionsdarstellung des sechsten Ausführungsbeispiels eines erfindungsgemäßen Platzhalters 10 und in den Fig. 11a- 11c eine Innenansicht des sechsten Ausführungsbeispiels eines erfindungsgemäßen Platzhalters 10 im geschlossenen Zustand, im geöffneten Zustand und im expandierten, also geöffneten und angehobenem Zustand dargestellt.

Dieses sechste Ausführungsbeispiel des erfindungsgemäßen Platzhalters 10 ist mit dem fünften Ausführungsbeispiel bis auf die folgenden Besonderheiten beschrieben:
- Die erste 21-1, 23-1 wie auch die zweite Teilfläche 21-2, 23-2 der oberen 21 wie auch der unteren Auflagefläche 23 weisen an der Kante 24-1, 24-2, 25-1, 25-1, an der sie sich im geschlossenen Zustand berühren, eine unregelmäßig ineinandergreifende Struktur 26 über die gesamte Länge der oberen 21 wie auch der unteren Auflagefläche 23 auf. Auch diese Struktur ist derart gestaltet, dass sie bei einer Expansion ein laterales Auseinanderdriften der jeweils ersten 21-1, 23-1 und zweiten Teilfläche 21-2, 23-2 ermöglicht. Obwohl die ineinandergreifende Struktur 26 nicht über die gesamte Breite der Auflageflächen 21, 23 im geschlossenen Zustand des Platzhalters 10 verläuft, entsteht auch hier im expandierten Zustand kein lateraler Spalt, die Spaltbreite eines gedachten lateralen Spalts senkrecht zur Richtung des lateralen Auseinanderdriftens ist also gleich Null. Und auch hier ist das obere 20 wie auch das untere Auflager 22 derart geformt, dass die ineinandergreifende Struktur 26 der ersten Teilfläche 21-1, 23-1 auf einer Auflagestruktur 44 im zur zweiten Teilfläche 21-2, 23-2 zugehörigem Teil des Auflagers 20, 22 und die ineinandergreifende Zahnstruktur 26 der zweiten Teilfläche 21-2, 23-2 auf einer Auflagestruktur 44 im zur ersten Teilfläche 21-1, 23-1 zugehörigem Teil des Auflagers 20, 22 beim lateralen Auseinanderdriften während der Expansion des Platzhalters 10 noch stabiler und sicherer aneinander gleiten kann.
- Das sechste Ausführungsbeispiel des erfindungsgemäßen Platzhalters 10 weist in seiner Draufsicht eine einseitig nierenförmige bzw. bogenförmige Gestalt auf, während die Gestalt auf der anderen Seite im weitesten Sinne quaderförmig mit abgerundeten Ecken ist. Es eignet sich deshalb besonders für einen bogenförmigen Implantationsweg 70, da insbesondere die ansonsten "aneckende" Seite des Platzhalters 10 bogenförmig gestaltet ist, jedoch ist seine Verwendung auch auf einem geraden Implantationsweg 70 nicht ausgeschlossen.

Sowohl in der Explosionsdarstellung der Fig. 10 als auch in den Ansichten des Innenlebens der Fig. 11a - 11c des sechsten Ausführungsbeispiels ist die zweiteilige Ausführung der Schraubspindel 31 mit einem Rechtsgewinde auf ihrer ersten Hälfte 31-1 und einem Linksgewinde auf ihrer zweiten Hälfte 31-2 sehr gut erkennbar.

Die Schraubspindel 31 dieses sechsten Ausführungsbeispiels eines erfindungsgemäßen Platzhalters weist eine Führungsstruktur 45 zwischen ihrer ersten 31-1 und zweiten Hälfte 31-2 auf, die in einem Rückhalteelement 46 drehbar, aber lateral nicht in ihrer Lage verschiebbar gelagert ist, wobei das Rückhalteelement 46 beweglich, insbesondere vertikal beweglich, aber wiederum lateral nicht in seiner Lage verschiebbar im oberen 20 und im unteren Auflager 22 gelagert ist, und damit die Expansionsvorrichtung in Bezug auf die Auflager zentriert.

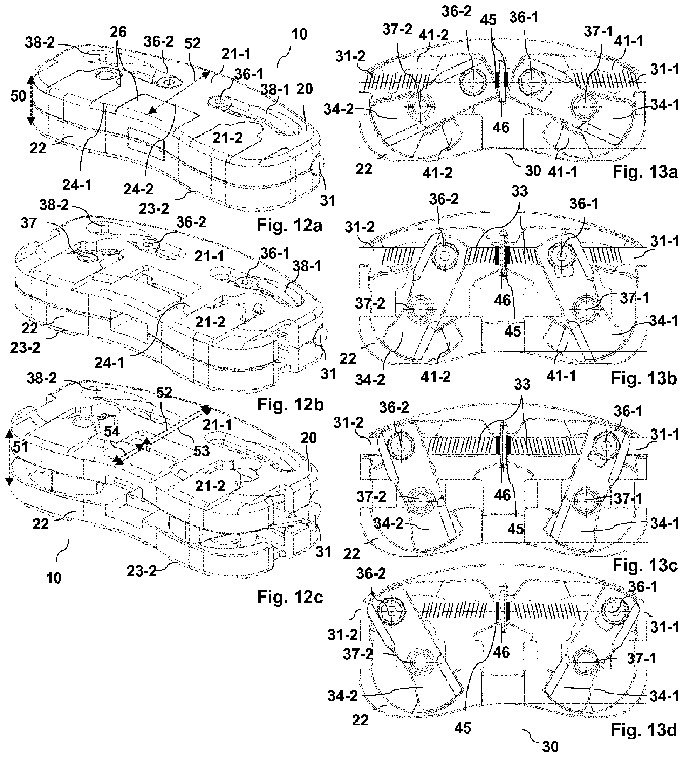

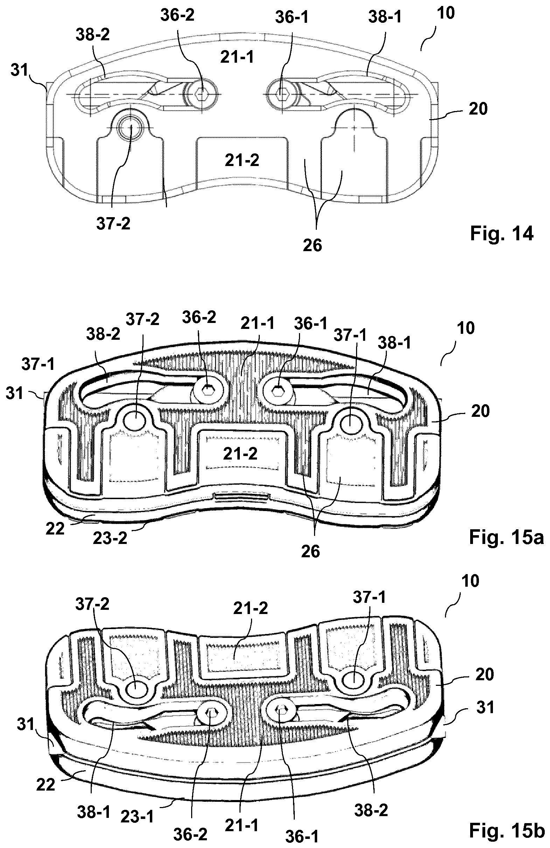

Die Fig. 12a - 12c zeigen schließlich ein siebtes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand in einer perspektivischen Ansicht. In der Fig. 14 ist eine Draufsicht und in den Fig. 15a und 15b zwei weitere perspektivische - im Vergleich zu den Fig. 12a-12c plastischer ausgestalteten Ansichten des siebten Ausführungsbeispiels eines erfindungsgemäßen Platzhalters 10 im geschlossenen Zustand dargestellt.

Auch hier sei für die Beschreibung des siebten Ausführungsbeispiels zunächst auf die Beschreibung des sechsten Ausführungsbeispiels des erfindungsgemäßen Platzhalters 10 verwiesen, von dem es sich in den folgenden Punkten unterscheidet:
- Das siebte Ausführungsbeispiel des erfindungsgemäßen Platzhalters 10 weist wieder eine rein nierenförmige Gestalt auf und ist folglich für eine Implantation auf einem bogenförmigen Implantationsweg 70 besonders geeignet.
- Wie in den Innenansichten des siebten Ausführungsbeispiels eines erfindungsgemäßen Platzhalters der Fig. 13a bis 13d im geschlossenen Zustand bei einer Stellung der als Distanzelemente dienenden Nocken 34-1, 34-2 zur Achse der Schraubspindel 31 von ca. 25°, im geöffneten Zustand bei einer Stellung der Nocken 34-1, 34-2 zur Achse der Schraubspindel 31 von ca. 65°, im geöffneten und teilweise angehobenen Zustand bei einer Stellung der Nocken 34-1, 34-2 zur Achse der Schraubspindel 31 von ca. 110° sowie im expandierten, also vollständig geöffneten und angehobenem Zustand bei einer Stellung der Nocken 34-1, 34-2 zur Achse der Schraubspindel 31 von ca. 120° ersichtlich ist, ist dieses Ausführungsbeispiels dasjenige, das die verschiedenen erfindungsgemäßen Merkmale zum größten Nutzen miteinander vereint:

Neben der ineinandergreifenden Struktur 26 der Teilflächen 21-1, 23-1, 21-2, 23-2 mit der Unterstützung von Auflagestrukturen 44 in den Auflagern 20, 22 der jeweils anderen Teilfläche 21-2, 23-2, 21-1, 23-1 und damit verbunden einem sicheren Gleiten während der Veränderung der lateralen Ausdehnung sowie eine Verhinderung eines durchgehenden lateralen Spaltes 27, einer nierenförmigen Gestalt zur Nutzung in einem bogenförmigen Implantationsweg 70, einer Anordnung der Schraubspindel 31 im Randbereich des Platzhalters 10 und der Nutzung zweier Distanzelemente 34-1, 34-2 in Form von Nocken, die spiegelbildlich arbeiten, wobei die Schraubspindel 31 wiederum eine Führungsstruktur 45 zwischen ihrer ersten 31-1 und zweiten Hälfte 31-2 aufweist, die in einem Rückhalteelement 46 drehbar, aber lateral nicht in ihrer Lage verschiebbar gelagert ist, und das Rückhalteelement 46 nur vertikal beweglich im oberen 20 und im unteren Auflager 22 gelagert ist, zeigt dieses siebte Ausführungsbeispiel des erfindungsgemäßen Platzhalters 10 eine Expansionsvorrichtung 30, die die Veränderung der lateralen Ausdehnung und des vertikalen Abstands in zwei voneinander unabhängig und frei definierten - und im Verlaufe der Expansion mehrfach variierten - Bewegungsverläufen mittels eines einzigen Antriebs ausführt: Ausgeführt ist dies mit nichtlinearen, gekrümmten Gleitflächen bzw. "Verschiebeflächen", die Gelenkflächen der Wirbelgelenke ähneln, sowie entsprechend geformten und als Gleitlöcher genutzten Langlöchern.

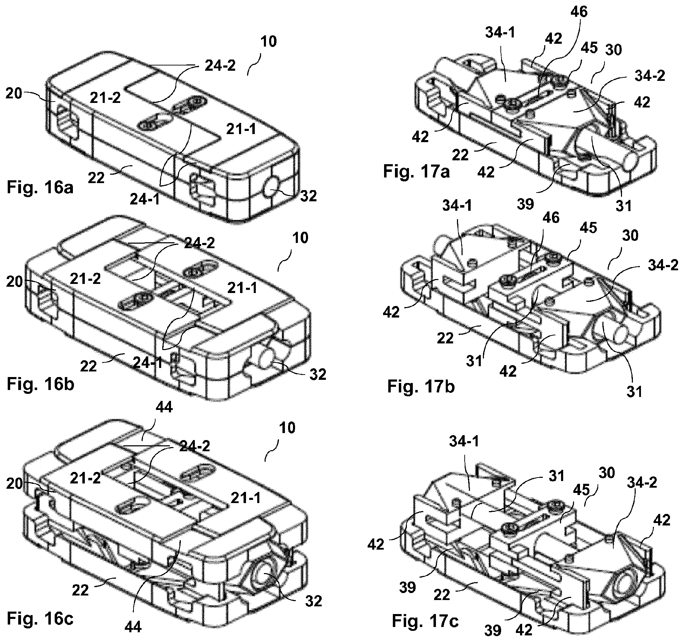

In den Fig. 16a - 16c wird ein achtes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand in einer perspektivischen Ansicht dargestellt. Die Fig. 17a - 17c zeigen eine entsprechende und ebenfalls perspektivische Innenansicht des achten Ausführungsbeispiels.

Dieser Platzhalter 10 umfasst wiederum ein oberes Auflager 20 mit einer oberen Auflagefläche 21 und ein unteres Auflager 22 mit einer unteren Auflagefläche 23, deren relative Lage zueinander veränderbar ist, wobei die obere 21 wie auch die untere Auflagefläche 23 jeweils eine erste 21-1, 23-1 und ein zweite Teilfläche 21-2, 23-2 aufweist, die sich in einem geschlossenen Zustand des Platzhalters 10 an einer Kante 24-1, 24-2, 25-1, 25-1 berühren. Der Platzhalter umfasst zudem eine Expansionsvorrichtung 30, mit der die Auflageflächen 21, 23 in ihrer lateralen Ausdehnung durch laterales Auseinanderdriften der ersten 21-1, 23-1 und zweiten Teilfläche 21-2, 23-2 wie auch in ihrem vertikalen Abstand zwischen oberer 21 und unterer Auflagefläche 23 zueinander veränderbar sind, so dass der Platzhalter 10 zwischen einem geschlossenen und einem expandierten Zustand einstellbar ist.

Dieses achte Ausführungsbeispiel des erfindungsgemäßen Platzhalters verfolgt wiederum ein Keilprinzip, das in den ersten drei Ausführungsbeispielen beschrieben wird, jedoch in abgewandelter Form: Die beweglichen Distanzelemente 34-1, 34-2 weisen wiederum eine Keilform auf, die aber nicht allein für die Veränderung des vertikalen Abstands von oberen 20 und unterem Auflager 22 zuständig ist. Vielmehr umfassen die beweglichen Distanzelemente 34-1, 34-2 hierzu an ihren Seitenflächen Führungselemente 42. Diese Führungselemente gleiten wiederum in oberen und unteren Führungsgleitflächen 38, 39 die unter den oberen Teilflächen 21-1, 21-2 des oberen Auflagers 20 sowie auf den unteren Teilflächen 23-1, 23-2 des unteren Auflagers 22 (also nach innen gerichtet und bei einer Sichtweise, dass "auf" eine äußere Lage wiederspiegelt, dann auch wieder "unter" den unteren Teilflächen 23-1, 23-2) angeordnet sind, so dass die an den Distanzelementen 34-1, 34-2 angeordneten Führungselemente 42 in diesen oberen und unteren Führungsgleitflächen 38, 39 gleiten können.

Die Führungsgleitflächen 38, 39 sind dabei - im Gegensatz zu den in anderen Ausführungsbeispielen an etwa ähnlichen Positionen erwähnten oberen und unteren bevorzugt dreidimensionalen Gleitflächen 40, 41, so ausgestaltet, dass sie die laterale Positionierung vollumfänglich mit übernehmen, also in ihrer Formung sehr scharf begrenzt sind und sowohl den Verlauf der lateralen und als auch der vertikalen Bewegungen genau abbilden. Die Expansionsvorrichtung 30 dieses achten Ausführungsbeispiels kann auf diese Art und Weise die Veränderung der lateralen Ausdehnung und des vertikalen Abstands in zwei voneinander unabhängig und frei definierbaren Bewegungsverläufen aber mittels eines einzigen Antriebs auszuführen: Lateraler und vertikaler Verlauf können durch entsprechende Formung frei in die Führungsgleitflächen 38, 39 kodiert werden. Weiteren (Gleit-)Flächen der Distanzelemente 34-1, 34-2 unterstützen diesen Bewegungsverlauf.

Die Distanzelemente 34-1, 34-2 bewegen sich - im Gegensatz zu den keilförmigen Distanzelementen der ersten drei Ausführungsbeispiele - auf einer Schraubspindel 31 von innen (um einen geschlossenen Zustand zu realisieren) nach außen, also voneinander weg, um einen expandierten Zustand zu realisieren. Im geschlossenen Zustand liegen die beiden keilförmigen Distanzelemente 34-1, 34-2 an einer ebenfalls auf der Schraubspindel 31 angeordneten Führungsstruktur 45 an, in der wiederum ein Rückhalteelement 46 auf der Schraubspindel 31 für die korrekte Positionierung der Führungsstruktur 45 zur Schraubspindel und sich in lateralen Langlöchern in den Teilflächen 21-1, 21-2, 23-1, 23-2 bewegende Strukturen für die korrekte Positionierung der Führungsstruktur 45 zum oberen 20 und unteren Auflager 22 sorgen.

Auch in diesem achten Ausführungsbeispiel des erfindungsgemäßen Platzhalters 10 weist die erste 21-1, 23-1 wie auch die zweite Teilfläche 21-2, 23-2 der oberen 21 wie auch der unteren Auflagefläche 23 an der Kante 24-1, 24-2, 25-1, 25-1, an der sie sich im geschlossenen Zustand berühren, eine ineinandergreifende Struktur mit zusätzlichen Auflagestrukturen 44 auf. Auch diese ineinandergreifende Struktur ist derart gestaltet, dass sie bei einer Expansion ein laterales Auseinanderdriften der ersten 21-1, 23-1 und zweiten Teilfläche 21-2, 23-2 ermöglicht, und im expandierten Zustand ein senkrecht zur Richtung des lateralen Auseinanderdriftens durch die oberen 21 wie auch die untere Auflagefläche 23 verlaufender lateraler Spalt 27 eine Spaltbreite 55 aufweist, die wesentlich kleiner ist als der Maximaldriftbetrag 54 - in Fall dieses achten Ausführungsbeispiels quasi gar nicht existent und damit Null ist. Im geschlossenen Zustand des Platzhalters 10 zeigen die ineinandergreifenden Strukturen der ersten und zweiten Teilfläche der oberen wie auch der unteren Auflageflächen ein Ineinandergreifen nach dem "Schlüssel-Schloss-Prinzip" über die gesamte Länge des Platzhalters, so dass im geschlossenen Zustand die obere wie auch die untere Auflagefläche als jeweils eine durchgehende Fläche wahrgenommen werden kann.

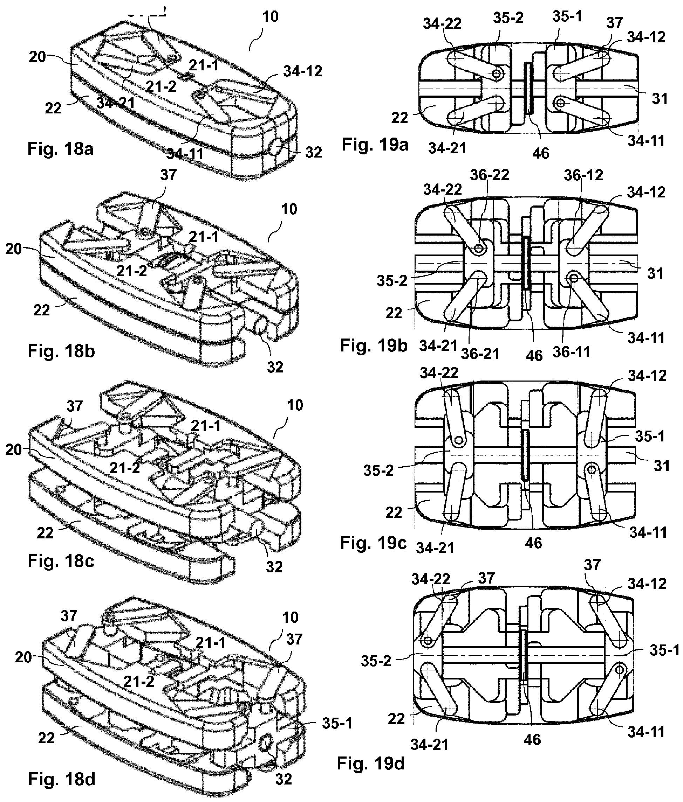

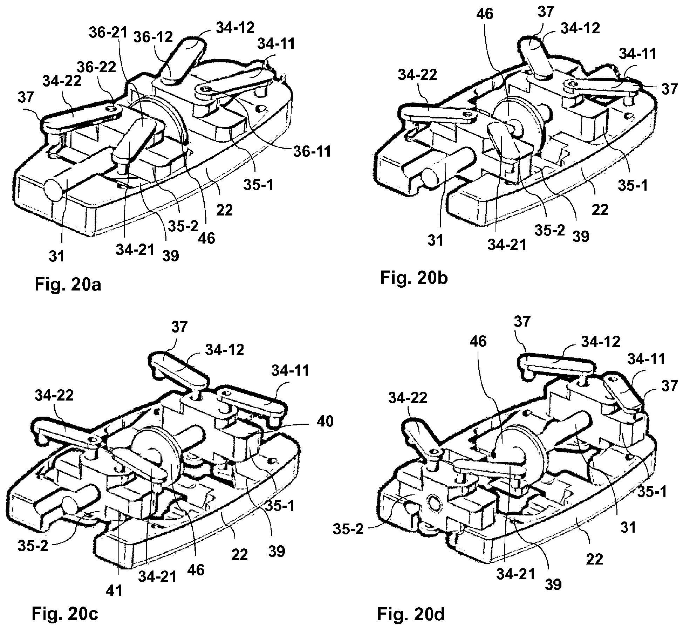

Die Fig. 18a - 18d zeigen ein neuntes Ausführungsbeispiel eines erfindungsgemäßen Platzhalters 10 für die Wirbelsäulenchirurgie im geschlossenen Zustand, im geöffneten Zustand, im expandierten, also geöffneten und angehobenen Zustand, sowie im expandierten und arretierten Zustand in einer perspektivischen Ansicht. In den Fig. 19a - 19d ist eine Innenansicht des neunten Ausführungsbeispiels im geschlossenen Zustand, im geöffneten Zustand, im expandierten, also geöffneten und angehobenen Zustand, sowie im expandierten und arretierten Zustand in einer Draufsicht dargestellt und die Fig. 20a -20d zeigen die Innenansicht des neunten Ausführungsbeispiels wiederum in einer perspektivischen Ansicht.

In diesem neunten Ausführungsbeispiel wurde die Zahl der beweglichen Distanzelemente 34-11, 34-12, 34-21, 34-22 gegenüber dem vierten bis siebten Ausführungsbeispiel verdoppelt. Jedes Distanzelement 34-11, 34-12, 34-21, 34-22 agiert als Doppelpaar, das lateral drehbar ist um eine fixe Achse 37 in der ersten Teilfläche 21-1, 23-1 des oberen 20 und unteren Auflagers 22 bzw. in der zweiten Teilfläche 21-2, 23-2 des oberen 20 und unteren Auflagers 22, sowie um eine bewegliche Achse 36-11, 36-12, 36-21, 36-22 die auf einer Gewindemutter 35-1, 35-2 angeordnet ist, wobei die Gewindemutter 35-1, 35-2 auf der Schraubspindel 31 von innen (um einen geschlossenen Zustand zu realisieren) nach außen, also voneinander weg, um einen expandierten Zustand zu realisieren, gleiten kann.

Dabei realisiert die Position der Gewindemutter 35-1, 35-2 auf der Schraubspindel 31 über die für diese Position entsprechend eingenommene Winkelposition der Distanzelement-Doppelpaare 34-11, 34-12, 34-21, 34-22 die laterale Ausdehnung des Platzhalters 10 und die spezielle Form der Gewindemutter 35-1, 35-2, die zu diesem Zwecke Gleitflächen 40, 41 aufweist, die auf Führungsgleitflächen 38, 39 in den ersten 21-1, 23-1 und zweiten Teilflächen 21-2, 23-2 des oberen 20 und unteren Auflagers 22 gleiten können, die vertikale Ausdehnung des Platzhalters 10.

Wiederum sorgte ein Rückhalteelement 46 auf der Schraubspindel 31 für die korrekte Positionierung der gesamten Expansionsvorrichtung 30 zum oberen 20 und unteren Auflager 22.

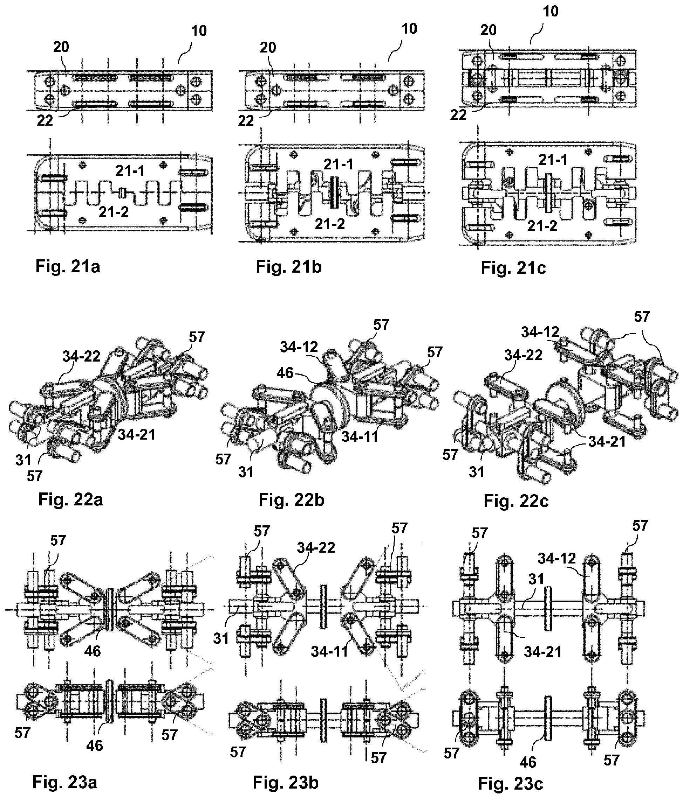

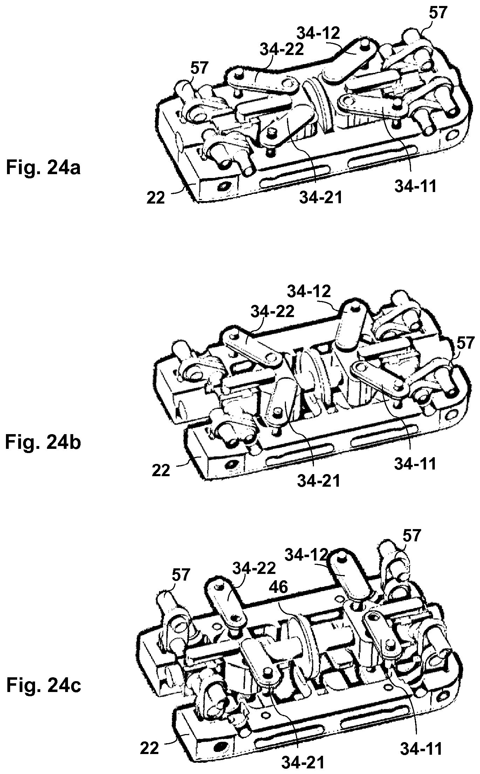

In den Fig. 21a - 21c wird ein zehntes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand in einer Seitenansicht und einer Draufsicht dargestellt, Die Fig. 22a - 22c zeigen eine Innenansicht der entsprechenden Zustände des zehnten Ausführungsbeispiels in einer perspektivischen Ansicht, die Fig. 23a - 23c eine Innenansicht des entsprechenden Zustands des zehnten Ausführungsbeispiels jeweils in einer Draufsicht und einer Seitenansicht. In den Fig. 24a - 24c ist eine weitere Innenansicht der jeweiligen Zustände des zehnten Ausführungsbeispiels in einer perspektivischen Ansicht dargestellt.

Dieses zehnte Ausführungsbeispiel entspricht in seinem Aufbau zunächst dem neunten Ausführungsbeispiel. Die vertikale Ausdehnung wird jedoch nicht durch eine Gleitflächen umfassende Gewindemutter 35-1, 35-2 realisiert, sondern die Gewindemutter 25-1, 25-2 umfasst hier jeweils eine Kniehebelstruktur 57, die sich in entsprechenden Führungsstrukturen an den Unterseiten der Teilflächen 21-1, 21-2, 23-1, 23-2 des oberen 20 und unteren 22 Auflagers befinden: Die Stellung der Gewindemutter 35-1, 35-2 auf der Schraubspindel 31 bestimmt dabei die Stellung der Kniehebelstruktur 57, deren Öffnungswinkel und damit die vertikale Ausdehnung des Platzhalters 10.

Das zehnte Ausführungsbeispiel weist des Weiteren auch wieder eine ineinandergreifende Struktur auf, die derart gestaltet ist, dass sie bei einer Expansion ein laterales Auseinanderdriften der ersten 21-1, 23-1 und zweiten Teilfläche 21-2, 23-2 ermöglicht, und im expandierten Zustand ein senkrecht zur Richtung des lateralen Auseinanderdriftens durch die oberen 21 wie auch die untere Auflagefläche 23 verlaufender lateraler Spalt 27 zwischen den ersten 21-1, 23-1 und zweiten Teilflächen 21-2, 23-2 eine Spaltbreite 55 aufweist, die wesentlich kleiner ist als der Maximaldriftbetrag 54.

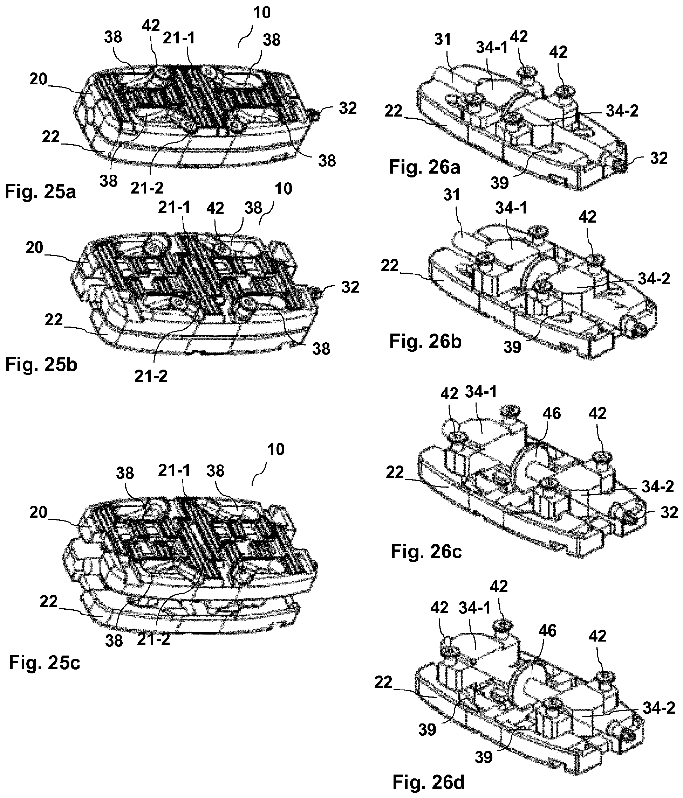

Die Fig. 25a - 25c zeigen ein elftes Ausführungsbeispiel eines erfindungsgemäßen Platzhalters 10 für die Wirbelsäulenchirurgie im geschlossenen Zustand, im geöffneten Zustand und im expandierten, also geöffneten und angehobenen Zustand in einer perspektivischen Ansicht. In den Fig. 26a - 26d wird eine Innenansicht des elften Ausführungsbeispiels eines erfindungsgemäßen Platzhalters 10 im geschlossenen Zustand, im geöffneten Zustand, im expandierten, also geöffneten und angehobenen Zustand, sowie im expandierten und arretierten Zustand in einer perspektivischen Ansicht dargestellt.

Dieses elfte Ausführungsbeispiel eines erfindungsgemäßen Platzhalters umfasst wiederum bewegliche Distanzelemente 34-1, 34-2, die sich auf der Schraubspindel 31 gegenläufig bewegen: In einem geschlossenen Zustand des Platzhalter 10 sind diese Distanzelemente 34-1, 34-2 im Inneren des Platzhalters 10 einer Führungsstruktur 45 mit Rückhalteelement 46 benachbart. Während der Expansion des Platzhalters 10 bewegen sich diese Distanzelemente 34-1, 34-2 voneinander weg nach außen. Das elfte Ausführungsbeispiel arbeitet dabei nach einer Kombination eines Keilprinzips mit einer Führung durch entsprechend geformte Langlöcher 38, 39 (die den Ablauf der lateralen Ausdehnung bestimmen), in denen Führungselemente 42 (hier in einer Form von Pilzkopfbolzen) der Distanzelemente 34-1, 34-2 gleiten, sowie zusätzliche Gleitflächen 40, 41 unter den Teilflächen 21-1, 21-2, 23-1, 23-2 des oberen 20 und unteren Auflagers 22 und, optional, auch in den Langlöchern 38, 39 selbst, durch die der Ablauf der vertikalen Ausdehnung des Platzhalters 10 bestimmt wird.

Auch das elfte Ausführungsbeispiel weist wiederum eine ineinandergreifende Struktur der ersten 21-1, 23-1 und zweiten Teilflächen 21-2, 23-2 des oberen 20 und unteren Auflagers 22 auf.

Dieses elfte Ausführungsbeispiel eines erfindungsgemäßen Platzhalters 10 zeigt eine besonders stabile Ausführungsform eines solchen Platzhalters 10.

Alle Ausführungsbeispiele sind so ausgestaltet, dass die Platzhalter in einfacher Art und Weise implantiert und dann entsprechend expandiert werden können, sie ermöglichen aber auch den entsprechenden umgekehrten Weg, wenn dies nötig ist: Sie können durch eine entsprechende Bewegung der Schraubspindel 31 in entgegengesetzter Drehrichtung wieder geschlossen werden und sind damit einfacher wieder der Wirbelsäule des Patienten entnehmbar.

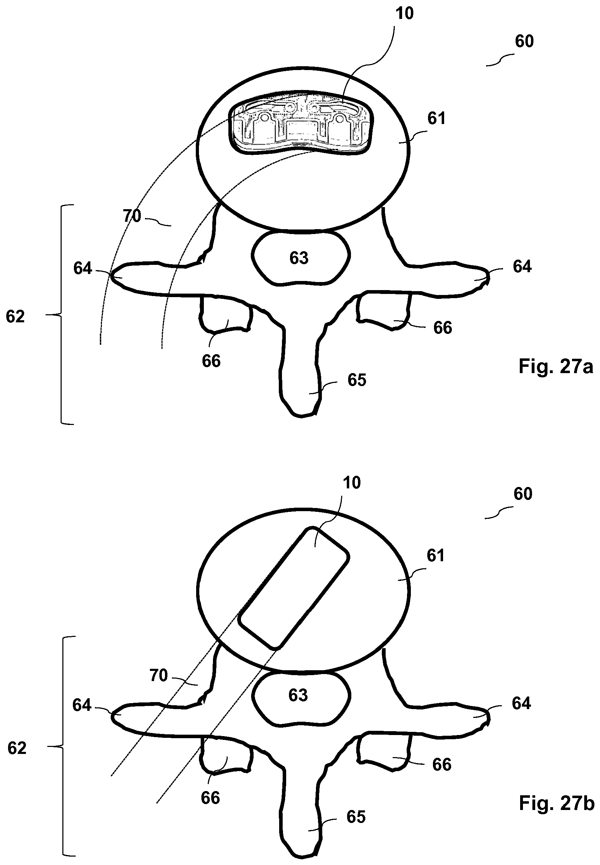

In den Fig. 27a und 27b sind zwei Varianten der Implantation des Platzhalters in einer Funktion als Zwischenwirbelkörperimplantat dargestellt:
Die Fig. 27a zeigt einen bogenförmigen Implantationsweg 70 eines nierenförmigen erfindungsgemäßen Platzhalters 10 hinein in ein Bandscheibenfach zwischen zwei Wirbelkörpern 61 eines Wirbels 60 der Wirbelsäule eines Patienten. Ein Wirbel 60 weist dabei einen Wirbelkörper 61 und einen Wirbelbogen 62 auf, der wiederum Querfortsätze 64, Dornenfortsatz 65 und Gelenkfortsätze 66 umfasst. In seinem Inneren befindet sich der Wirbelkanal 63, der auf keinen Fall geschädigt werden darf, da hier neurale Strukturen hindurch verlaufen. In der Fig. 27b ist hingegen ein gerader Implantationsweg 70 eines Platzhalters 10 mit quaderförmiger Grundform in eben dieses Bandscheibenfach dargestellt.

Dabei ist aus dem Vergleich der Fig. 27a und 27b leicht erkennbar, dass ein minimalinvasiv nutzbarer bogenförmiger Implantationsweg 70 - wie auch ein minimalinvasiv nutzbarer gerader Implantationsweg 70 - durch den Wirbelbogen 62 hindurchführt, der bogenförmige Implantationsweg 70 der Fig. 27a jedoch den Wirbelkanal 63 relativ weitläufig meidet, während ein gerader Implantationsweg 70, wie in der Fig. 27b gezeigt, gefährlich nahe am Wirbelkanal 63 entlang verläuft.

Zudem ist der nierenförmige Platzhalter 10, der über den bogenförmigen Implantationsweg 70 implantiert wurde, ventral sehr leicht symmetrisch in das Bandscheibenfach einzubringen, während ein gerader Implantationsweg 70 zu einer nichtsymmetrischen Lage des Platzhalters 10 im Bandscheibenfach führt.

Die Fig. 27a macht zudem noch einmal deutlich, dass ein nierenförmiger erfindungsgemäßer Platzhalter 10 im Vergleich zu einem quaderförmigen erfindungsgemäßen Platzhalter 10 wesentlich leichter über einen bogenförmigen Implantationsweg 70 an seinen Wirkungsort zu verbringen ist.

Das Verfahren zur Implantation des Platzhalters 10 über den bogenförmigen oder den geraden Implantationsweg 70 wurde bereits oben beschrieben.

Abschließend sei ganz besonders darauf hingewiesen, dass die voranstehend erörterten

Ausführungsbeispiele lediglich zur Beschreibung der beanspruchten Lehre dienen, diese jedoch nicht auf die Ausführungsbeispiele einschränken. Insbesondere könnten die oben beschriebenen Ausführungsbeispiele - soweit möglich - miteinander kombiniert werden: Die Vielzahl der hier gezeigten Ausführungsbeispiele des erfindungsgemäßen Platzhalters 10 zeigt eine Auswahl des Einsatzes der erfindungsgemäßen Merkmale allein wie auch in Kombination: Wie hier gezeigt wurde, erfüllt jedes Merkmal allein bereits die Aufgabe der vorliegenden Erfindung. Durch die Kombination der erfindungsgemäßen Merkmale entsteht dann nochmals ein zusätzlicher Nutzen.

## Patentansprüche

1. Platzhalter (10) für die Wirbelsäulenchirurgie, der umfasst:
- ein oberes Auflager (20) mit einer oberen Auflagefläche (21) und ein unteres Auflager (22) mit einer unteren Auflagefläche (23), deren relative Lage zueinander veränderbar ist, wobei die obere (21) wie auch die untere Auflagefläche (23) jeweils eine erste (21-1, 23-1) und ein zweite Teilfläche (21-2, 23-2) aufweist, die sich in einem geschlossenen Zustand des Platzhalters (10) an einer Kante (24-1, 24-2, 25-1, 25-1) berühren, und
- eine Expansionsvorrichtung (30), mit der die Auflageflächen (21, 23) in ihrer lateralen Ausdehnung durch laterales Auseinanderdriften der ersten (21-1, 23-1) und zweiten Teilfläche (21-2, 23-2) bis zu einem Maximaldriftbetrag (54) zwischen einer minimalen lateralen Ausdehnung (52) und einer maximalen lateralen Ausdehnung (53) wie auch in ihrem vertikalen Abstand zwischen einer Minimalhöhe (50) und einer Maximalhöhe (51) des Platzhalters (10) zueinander veränderbar sind, so dass der Platzhalter (10) zwischen einem geschlossenen und einem expandiertem Zustand einstellbar ist,
- **dadurch gekennzeichnet, dass** die Expansionsvorrichtung (30), die einen einzigen Antrieb aufweist, eingerichtet ist, die Veränderung der lateralen Ausdehnung und des vertikalen Abstands in zwei voneinander unabhängig und frei definierbaren und im Platzhalter kodierten Bewegungsverläufen, aber mittels dieses einzigen Antriebs, mittels Bedienung immer desselben Stellelements, auszuführen, wobei die Kodierung der Bewegungsverläufe im Platzhalter (10) durch Freiformflächen als dreidimensionale Gleitflächen oder Führungsgleitflächen an Innenseiten oder Kanten des oberen (20) wie auch des unteren Auflagers (22), auf im Inneren verwendeten und als Gleitflächen genutzten Distanzelementen, in als Gleitlöcher eingesetzten Langlöchern mit frei bestimmbarer Form oder in drehbaren Strukturen mit unterschiedlicher Zahnteilung erfolgt.

2. Platzhalter nach Anspruch 1, dessen Expansionsvorrichtung (30) sowohl im geschlossenen wie auch im expandierten Zustand im Inneren eines von den oberen (21) und unteren Auflageflächen (23) aufgespannten Volumens verbleibt.

3. Platzhalter (10) nach Anspruch 1 oder 2, **dadurch gekennzeichnet, dass** die erste (21-1, 23-1) wie auch die zweite Teilfläche (21-2, 23-2) der oberen (21) wie auch der unteren Auflagefläche (23) an der Kante (24-1, 24-2, 25-1, 25-1), an der sie sich im geschlossenen Zustand berühren, eine ineinandergreifende Struktur (26) aufweist, die derart gestaltet ist, dass sie bei einer Expansion ein laterales Auseinanderdriften der ersten (21-1, 23-1) und zweiten Teilfläche (21-2, 23-2) ermöglicht, und im expandierten Zustand ein senkrecht zur Richtung des lateralen Auseinanderdriftens durch die oberen (21) wie auch die untere Auflagefläche (23) verlaufender lateraler Spalt (27) eine Spaltbreite (55) aufweist, die kleiner ist als der Maximaldriftbetrag (54) und größer gleich Null beträgt.

4. Platzhalter nach Anspruch 3, dessen oberes (20) wie auch unteres Auflager (22) derart geformt ist, dass beim lateralen Auseinanderdriften die ineinandergreifende Struktur (26) der ersten Teilfläche (21-1, 23-1) auf einer Auflagestruktur (44) im zur zweiten Teilfläche (21-2, 23-2) zugehörigen Teil des Auflagers (20, 22) und die ineinandergreifende Struktur (26) der zweiten Teilfläche (21-2, 23-2) auf einer Auflagestruktur (44) im zur ersten Teilfläche (21-1, 23-1) zugehörigen Teil des Auflagers (20, 22) lagernd gleitet.

5. Platzhalter (10) nach einem der Ansprüche 1 bis 4, wobei die Expansionsvorrichtung (30) zur Einstellung des Platzhalters (10) zwischen dem geschlossenen und dem expandierten Zustand eine Schraubspindel (31) mit einem Schraubenkopf (32) enthält, mit der die Veränderung der lateralen Ausdehnung wie auch des vertikalen Abstands steuerbar ist.

6. Platzhalter (10) nach Anspruch 5, der in einer beliebigen lateralen Schnittebene keine Symmetrien entlang einer Achse, die parallel der Achse der Schraubspindel (31) verläuft, aufweist.

7. Platzhalter (10) nach Anspruch 5 oder 6, dessen Expansionsvorrichtung (30) des Weiteren ein bewegliches Distanzelement (34) umfasst,
- das an einer Position auf dem Distanzelement (34) lateral drehbar um eine bewegliche Achse (36) gelagert ist, die mittels einer in die bewegliche Achse (36) integrierte und auf einem Gewinde (33) der Schraubspindel (31) laufenden Gewindemutter (35) entlang der Schraubspindel (31) verschiebbar ist, wobei die bewegliche Achse (36) direkt oder indirekt in einem oberen Langloch (38) in der ersten Teilfläche (21-1) des oberen Auflagers (20) und in einem unterem Langloch (39) in der ersten Teilfläche (23-1) des unteren Auflagers (22) laufend angeordnet ist,
- das des Weiteren lateral drehbar ist um eine fixe Achse (37), die in der zweiten Teilfläche (21-2) des oberen Auflagers (20) und in der zweiten Teilfläche (23-2) des unteren Auflagers (22) lateral ortsunveränderlich gelagert ist, und
- das an einem ersten und/oder einem zweiten Ende frei gleitend auf einer oberen dreidimensionalen Gleitfläche (40) unter der ersten (21-1) und/oder der zweiten Teilfläche (21-2) des oberen Auflagers (20) sowie auf einer unteren dreidimensionalen Gleitfläche (41) über der ersten (23-1) und/oder der zweiten Teilfläche (23-2) des unteren Auflagers (22) gelagert ist, wobei die obere dreidimensionale Gleitfläche (40) und die untere dreidimensionale Gleitfläche (41) so zueinander geformt sind, dass das erste und/oder zweite Ende des Distanzelements (34) für jeden Drehwinkel des Distanzelements (34) um die fixe Achse (37) eine definierte Position auf der oberen (40) wie auch auf der unteren dreidimensionalen Gleitfläche (41) einnimmt, deren absolute Lage und die dazu korrespondierende Position der beweglichen Achse (36) in den entsprechend geformten Langlöchern (38, 39) die laterale Ausdehnung des Platzhalters (10) und deren Abstand der oberen (40) und der unteren dreidimensionalen Gleitfläche (41) zueinander im geschlossenen Zustand die Höhe des Platzhalters (10) bestimmt.

8. Platzhalter (10) nach Anspruch 5 oder 6, dessen Expansionsvorrichtung (30) des Weiteren ein bewegliches Distanzelement (34) umfasst,
- das entlang der Schraubspindel (31) bewegbar ist und vier Führungselemente (42) aufweist, die jeweils in einem ersten oberen Langloch und/oder unter einer ersten oberen Führungsgleitfläche (38) in der ersten Teilfläche (21-1) und in einem zweiten oberen Langloch und/oder unter einer zweiten oberen Führungsgleitfläche (38) der zweiten Teilfläche (21-2) des oberen Auflagers (20) und in einem ersten unteren Langloch und/oder auf einer ersten unteren Führungsgleitfläche (39) der ersten Teilfläche (23-1) und in einem zweiten unteren Langloch und/oder auf einer zweiten unteren Führungsgleitfläche (39) der zweiten Teilfläche (23-2) des unteren Auflagers (22) verschiebbar sind,
- wobei das Distanzelement (34) obere und untere, bevorzugt dreidimensionale, Gleitflächen (40, 41) aufweist, und
- wobei die obere Gleitfläche (40) und die untere Gleitfläche (41) auf dem Distanzelement (34) so zueinander geformt sind, und die Langlöcher und/oder Führungsgleitflächen (38, 39) in jeder der Teilflächen des oberen und unteren Auflagers so geformt und angeordnet sind, dass für eine definierte Position, die das Distanzelement (34) auf der Schraubspindel (31) einnimmt, die dazu korrespondierende Position der Führungselemente (42) in den entsprechend geformten Langlöchern und/oder Führungsgleitflächen (38, 39) die laterale Ausdehnung des Platzhalters (10) und deren Abstand der oberen (40) und der unteren Gleitfläche (41) zueinander auf dem Distanzelement (34) an einer Berührungskante (43) und/oder an einer der Position des Distanzelements (34) zugeordneten Position der oberen (38) und unteren Führungsgleitflächen (39) des oberen (20) und des unteren Auflagers (22) die Höhe des Platzhalters (10) bestimmt.

9. Platzhalter (10) nach Anspruch 5 oder 6, dessen Expansionsvorrichtung (30) des Weiteren ein bewegliches Doppelpaar von Distanzelementen (34-11, 34-12) umfasst,
- das an einer Position auf jedem Distanzelement (34-11, 34-12) des Doppelpaars jeweils lateral drehbar um eine bewegliche Achse (36-11, 36-12) gelagert ist, wobei die beweglichen Achsen (36-11, 36-12) auf einer Gewindemutter (35) angeordnet sind, und mittels der auf einem Gewinde (33) der Schraubspindel (31) laufenden Gewindemutter (35) entlang der Schraubspindel (31) verschiebbar sind,
- wobei das erste Distanzelement (34-11) des Doppelpaars lateral drehbar ist um eine fixe Achse (37), die in der ersten Teilfläche (21-1) des oberen Auflagers (20) und in der ersten Teilfläche (23-1) des unteren Auflagers (22) lateral ortsunveränderlich gelagert ist, und das zweite Distanzelement (34-12) des Doppelpaars lateral drehbar ist um eine fixe Achse (37), die in der zweiten Teilfläche (21-2) des oberen Auflagers (20) und in der zweiten Teilfläche (23-2) des unteren Auflagers (22) lateral ortsunveränderlich gelagert ist, und- wobei die auf dem Gewinde (33) der Schraubspindel (31) laufende Gewindemutter (35) derart ausgebildet ist, dass sie Gleitflächen (40, 41) oder eine doppelte Kniehebelstruktur (57) zur ersten (21-1) und zweiten Teilfläche (21-2) des oberen Auflagers (20) und zur ersten (23-1) und zweiten Teilfläche (23-2) des unteren Auflagers (22) umfasst, die auf Führungsgleitflächen (38) der ersten (21-1) und zweiten Teilfläche (21-2) des oberen Auflagers (20) und auf Führungsgleitflächen (39) der ersten (23-1) und zweiten Teilfläche (23-2) des unteren Auflagers (22) frei gleitend gelagert sind,
- und die Winkelposition zwischen dem ersten Distanzelement (34-11) und dem zweiten Distanzelement (34-12) die laterale Ausdehnung des Platzhalters (10) und ein Abstand der oberen (40) und der unteren Gleitfläche (41) zueinander oder eine Stellung der doppelten Kniehebelstruktur (57) an einer Berührungskante (43) und/oder an einer der Position der Gewindemutter (35) zugeordneten Position der oberen (38) und unteren Führungsgleitflächen (39) des oberen (20) und des unteren Auflagers (22) die Höhe des Platzhalters (10) bestimmt.

10. Platzhalter (10) nach einem der Ansprüche 7 bis 9, wobei die Schraubspindel (31) auf ihrer zweiten Hälfte (31-2) ein Gewinde (33) aufweist, das gegenläufig zu einem Gewinde (33) auf der ersten Hälfte (31-1) der Schraubspindel (31) ist, und die Expansionsvorrichtung (30) ein erstes Distanzelement (34-1) oder ein erstes Doppelpaar von Distanzelementen, das die erste Hälfte (31-1) der Schraubspindel nutzt und ein zweites Distanzelement (34-2) oder ein zweites Doppelpaar von Distanzelementen, das die zweite Hälfte (31-2) der Schraubspindel (31) nutzt, umfasst, wobei erstem (34-1) und zweitem Distanzelement oder Doppelpaar von Distanzelementen (34-2) erste (40-1, 41-1) bzw. zweite obere und untere dreidimensionale Gleitflächen (40-2, 41-2) und entsprechende obere (38-1, 38-2) und untere Langlöcher (39-1, 39-2) und/oder Führungsgleitflächen zugeordnet sind.

11. Platzhalter (10) nach einem der Ansprüche 7 bis 10, mit einen ersten und einem zweiten Distanzelement oder Doppelpaar von Distanzelementen, wobei das erste (34-1) und das zweite Distanzelement (34-2) oder Doppelpaar von Distanzelementen zueinander spiegelbildlich arbeitend ausgelegt und angeordnet sind, oder wobei das erste (34-1) und das zweite Distanzelement (34-2) oder Doppelpaar von Distanzelementen zueinander gleichläufig arbeitend ausgelegt und angeordnet sind.

12. Platzhalter (10) nach Anspruch 10 oder 11, dessen Schraubspindel (31) eine Führungsstruktur (45) zwischen erster (31-1) und zweiter Hälfte (31-2) und/oder zwischen erstem (34-1) und zweitem Distanzelement (34-2) oder Doppelpaar von Distanzelementen aufweist, die in einem Rückhalteelement (46) drehbar, aber lateral nicht in ihrer Lage verschiebbar gelagert ist, wobei das Rückhalteelement (46) beweglich, insbesondere vertikal beweglich, aber wiederum lateral nicht in seiner Lage verschiebbar im oberen (20) und im unteren Auflager (22) gelagert ist.

13. Platzhalter (10) nach einem der Ansprüche 7 bis 12, wobei die Form und Lage der dreidimensionalen Gleitflächen (40-1, 40-2, 41-2, 41-2) und die Form und Lage des Langlochs (38-1, 38-2, 39-1, 39-2) und/oder der Führungsgleitflächen nach einem individuell benötigten Expansionsverhalten gestaltet sind.

14. Platzhalter (10) nach einem der Ansprüche 1 bis 13, der in einer Draufsicht eine nierenförmige Gestalt aufweist.

15. Platzhalter (10) nach einem der Ansprüche 1 bis 14, der eine Minimalhöhe (50) von größer oder gleich 7mm im geschlossenen Zustand und eine Maximalhöhe (51) von kleiner oder gleich 14mm im expandierten Zustand sowie eine laterale Ausdehnung (52, 53) von größer oder gleich 13mm im geschlossenen Zustand aufweist.

## Claims

1. Placeholder (10) for spinal surgery, which comprises:
- an upper support (20) with an upper support surface (21) and a lower support (22) with a lower support surface (23), the relative position of which to one another can be changed, wherein the upper (21) as well as the lower support surface (23) in each case has a first (21-1, 23-1) and a second sub-surface (21-2, 23-2), which touches an edge (24-1, 24-2, 25-1, 25-1) in a closed state of the placeholder (10), and
- an expansion device (30), by means of which the support surfaces (21, 23) can be varied in their lateral extension by a lateral drifting apart of the first (21-1, 23-1) and second sub-surface (21-2, 23-2) up to a maximum drift amount (54) between a minimum lateral extension (52) and a maximum lateral extension (53) as well as in the vertical distance between a minimum height (50) and a maximum height (51) of the placeholder (10), so that the placeholder (10) can be adjusted between a closed and an expanded state,
- **characterised in that** the expansion device (30), containing a single drive, is configured to carry out the change in the lateral extension and the vertical distance in two movement courses which are independent of one another and freely definable and coded in the placeholder, but by means of this single drive, by means of operation of always the same adjusting element, wherein the coding of the movement courses in the placeholder (10) is achieved by free-form surfaces as three-dimensional sliding surfaces or guide sliding surfaces on the inner sides or edges of the upper (20) and lower (22) supports, on spacer elements used inside and serving as sliding surfaces, in elongated holes used as sliding holes with freely determinable shape or in rotatable structures with different tooth spacing.

2. Placeholder according to claim 1, whose expansion device (30) remains inside a volume spanned by the upper (21) and lower support surfaces (23) both in the closed and in the expanded state.

3. Placeholder according to claim 1 or 2, **characterised in that** the first (21-1, 23-1) as well as the second sub-surface (21-2, 23-2) of the upper (21) as well as the lower support surface (23) comprise an interengaging structure (26) on the edge (24-1, 24-2, 25-1, 25 -1), at which they touch in the closed state, which is designed in such a way that, in the event of an expansion, it permits a lateral drifting apart of the first (21-1, 23-1) and second sub-surfaces (21-2, 23-2), and, in the expanded state, a lateral gap (27) running perpendicular to the direction of the lateral drifting apart through the upper (21) and the lower support surface (23) has a gap width (55), which is smaller than the maximum drift amount (54) and bigger or equal zero.

4. Placeholder according to claim 3, the upper (20) and the lower support (22) of which are shaped in such a way that, during the lateral drifting part, the interengaging structure (26) of the first sub-surface (21 -1, 23-1) slides mounted on a support structure (44) in the part of the support (20, 22) associated with the second sub-surface (21-2, 23-2) and the interengaging structure (26) of the second sub-surface (21-2, 23-2) slides mounted on a support structure (44) in the part of the support (20, 22) associated with the first sub-surface (21-1, 23-1).

5. Placeholder (10) according to any one of claims 1 to 4, wherein the expansion device (30) for setting the placeholder (10) between the closed and the expanded state contains a screw spindle (31) with a screw head (32), by means of which the change in the lateral extension as well as the vertical distance can be controlled.

6. Placeholder (10) according to claim 5, which has no symmetries along an axis, which runs parallel to the axis of the screw spindle (31), in any lateral cutting plane.

7. Placeholder (10) according to claim 5 or 6, the expansion device (30) of which further comprises a movable spacer element (34),
- which is mounted in a position on the spacer element (34) so as to be laterally rotatable about a movable axis (36), which can be displaced along the screw spindle (31) by means of a threaded nut (35) which is integrated into the movable axis (36) and runs on a thread (33) of the screw spindle (31), wherein the movable axis (36) is arranged running directly or indirectly in an upper elongated hole (38) in the first sub-surface (21-1) of the upper support (20) and in a lower elongated hole (39) in the first sub-surface (23-1) of the lower support (22),
- which is further laterally rotatable about a fixed axis (37), which is mounted in the second sub-surface (21-2) of the upper support (20) and in the second sub-surface (23-2) of the lower support (22) in a laterally positionally invariable manner, and
- which is mounted on a first and/or a second end in a freely sliding manner on an upper three-dimensional sliding surface (40) below the first (21-1) and/or the second sub-surface (21-2) of the upper support (20) and on a lower three-dimensional sliding surface (41) above the first (23-1) and/or the second sub-surface (23-2) of the lower support (22), wherein the upper three-dimensional sliding surface (40) and the lower three-dimensional sliding surface (41) are shaped with respect to one another such that the first and/or second end of the spacer element (34) assumes a defined position on the upper (40) as well as on the lower three-dimensional sliding surface (41) for each angle of rotation of the spacer element (34) about the fixed axis (37), the absolute position of which and the corresponding position of the movable axis (36) in the correspondingly shaped elongated holes (38, 39) determines the lateral extension of the placeholder (10) and the distance of which between the upper (40) and the lower three-dimensional sliding surface (41) from each other in the closed state determines the height of the placeholder (10).

8. Placeholder (10) according to claim 5 or 6, the expansion device (30) of which further comprises a movable spacer element (34),
- which can be moved along the screw spindle (31) and has four guide elements (42), which are in each case displaceable in a first upper elongated hole and/or below a first upper guide sliding surface (38) in the first sub-surface (21-1) and in a second upper elongated hole and/or below a second upper guide sliding surface (38) of the second sub-surface (21-2) of the upper support (20) and in a first lower elongated hole and/or on a first lower guide sliding surface (39) of the first sub-surface (23-1) and in a second lower elongated hole and/or on a second lower guide sliding surface (39) of the second sub-surface (23-2) of the lower support (22),
- wherein the spacer element (34) comprises upper and lower, preferably three-dimensional, sliding surfaces (40,41), and
- wherein the upper sliding surface (40) and the lower sliding surface (41) on the spacer element (34) are shaped in respect to one another, and the elongated holes and/or guide sliding surfaces (38, 39) are shaped and arranged in each of the sub-surfaces of the upper and lower supports in such a way that, for a defined position, which the spacer element (34) assumes on the screw spindle (31), the corresponding position of the guide elements (42) in the correspondingly shaped elongated holes and/or guide sliding surfaces (38, 39) determines the lateral extension of the placeholder (10) and the distance of which between the upper (40) and lower sliding surfaces (41) from one another on the spacer element (34) at a touching edge (43) and/or at a position of the upper (38) and lower guide sliding surfaces (39) of the upper (20) and of the lower support (22), associated with the position of the spacer element (34), determines the height of the placeholder (10).

9. Placeholder (10) according to claim 5 or 6, the expansion device (30) of which further comprises a movable double pair of spacer elements (34-11, 34-12),
- which is mounted in a position on each spacer element (34-11, 34-12) of the double pair in each case in a laterally rotatable manner about a movable axis (36-11, 36-12), wherein the movable axes (36-11, 36-12) are arranged on a threaded nut (35), and can be displaced along the screw spindle (31) by means of the threaded nut (35) running on a thread (33) of the screw spindle (31),
- wherein the first spacer element (34-11) of the double pair is laterally rotatable about a fixed axis (37), which is mounted in the first sub-surface (21-1) of the upper support (20) and in the first sub-surface (23-1) of the lower support (22) in a laterally positionally invariable manner, and the second spacer element (34-12) of the double pair is laterally rotatable about a fixed axis (37), which is mounted in the second sub-surface (21-2) of the upper support (20) and in the second sub-surface (23-2) of the lower support (22) in a laterally positionally invariable manner, and
- wherein the threaded nut (35) running on the thread (33) of the screw spindle (31) is designed in such a way that it comprises sliding surfaces (40, 41) or a double toggle lever structure (57) for the first (21 -1) and second sub-surfaces (21 -2) of the upper support (20) and for the first (23-1) and second sub-surfaces (23-2) of the lower support (22), which are mounted in a freely sliding manner on guide sliding surfaces (38) of the first (21-1) and second sub-surfaces (21 -2) of the upper support (20) and on guide sliding surfaces (39) of the first (23-1) and second sub-surfaces (23 -2) of the lower support (22),
- and the angular position between the first spacer element (34-11) and the second spacer element (34-12) determines the lateral extension of the placeholder (10) and a distance of the upper (40) and lower sliding surface (41) relative to one another or a position of the double toggle lever structure (57) at a touching edge (43) and/or at a position of the upper (38) and lower guide sliding surfaces (39) of the upper (20) and of the lower support (22) associated with the position of the threaded nut (35) determines the height of the placeholder (10).

10. Placeholder (10) according to any one of claims 7 to 9, wherein the screw spindle (31) comprises a thread (33) on its second half (31-2), which runs in the opposite direction to a thread (33) on the first half (31-1) of the screw spindle (31), and the expansion device (30) comprises a first spacer element (34-1) or a first double pair of spacer elements, which uses the first half (31-1 ) of the screw spindle, and a second spacer element (34-2) or a second double pair of spacer elements, which uses the second half (31-2) of the screw spindle (31), wherein first (40-1, 41-1) or second upper and lower three-dimensional sliding surfaces (40-2, 41-2) and corresponding upper (38-1, 38-2) and lower elongated holes (39-1, 39-2) and/or guide sliding surfaces are associated with first (34-1) and second spacer element or double pair of spacer elements (34-2).

11. Placeholder (10) according to any one of claims 7 to 10, having a first and a second spacer element or double pair of spacer elements, wherein the first (34-1) and the second spacer element (34-2) or double pair of spacer elements are designed and arranged working in a mirror-inverted manner with respect to one another, or wherein the first (34-1) and the second spacer element (34-2) or double pair of spacer elements are designed and arranged working in opposite directions to one another.

12. Placeholder (10) according to claim 10 or 11, the screw spindle (31) of which comprises a guide structure (45) between the first (31-1) and the second half (31-2) and/or between the first (34-1) and the second spacer element (34-2) or double pair of spacer elements, which is mounted in a retaining element (46) so as to be rotatable but laterally not displaceable in its position, wherein the retaining element (46) is mounted movably, in particular vertically movably, but in turn is laterally not displaceable in its position in the upper support (20) and in the lower support (22).

13. Placeholder (10) according to any one of claims 7 to 12, wherein the shape and position of the three-dimensional sliding surfaces (40-1, 40-2, 41-2, 41-2) and the shape and position of the elongated hole (38-1, 38-2, 39-1, 39-2) and/or the guide sliding surfaces are designed according to an individually required expansion behaviour.

14. Placeholder (10) according to any one of claims 1 to 13, which, in a top view, has a kidney-shaped form.

15. Placeholder (10) according to any one of claims 1 to 14, which has a minimum height (50) of greater than or equal to 7 mm in the closed state and a maximum height (51) of less than or equal to 14 mm in the expanded state and a lateral extension (52, 53) of greater than or equal to 13 mm in the closed state.

## Revendications

1. Écarteur (10) pour la chirurgie de la colonne vertébrale, comprenant :
- un support supérieur (20) avec une surface d'appui supérieure (21) et un support inférieur (22) avec une surface d'appui inférieure (23), dont la position relative l'un par rapport à l'autre est modifiable, la surface d'appui supérieure (21) ainsi que la surface d'appui inférieure (23) présentant chacune une première (21-1, 23-1) et une deuxième surface partielle (21-2, 23-2) qui, lorsque l'écarteur (10) est à l'état fermé, se touchent au niveau d'un bord (24-1, 24-2, 25-1, 25-1), et
- un dispositif d'expansion (30) avec lequel les surfaces d'appui (21, 23) sont déployées, l'une par rapport à l'autre, latéralement par écartement latéral de la première (21-1, 23-1) et de la deuxième surface partielle (21-2, 23-2) jusqu'à une valeur d'écartement maximale (54) entre une extension latérale minimale (52) et une extension latérale maximale (53), ainsi que dans leur distance verticale entre une hauteur minimale (50) et une hauteur maximale (51) de l'écarteur (10), de sorte que l'écarteur (10) peut être réglé entre un état fermé et un état expansé,
- **caractérisé en ce que** le dispositif d'expansion (30), qui comporte un seul entraînement, est conçu pour effectuer la modification de l'extension latérale et de la distance verticale selon deux courses de mouvement indépendantes l'une de l'autre, librement définissables et codées dans l'écarteur, mais au moyen de ce seul entraînement, en actionnant toujours le même élément de commande, le codage des courses de mouvement dans l'écarteur (10) étant réalisé par des surfaces de forme libre sous forme de surfaces de glissement ou de surfaces de glissement de guidage tridimensionnelles sur les faces intérieures ou les arêtes du support supérieur (20) ainsi que du support inférieur (22), sur des éléments d'écartement utilisés à l'intérieur et servant de surfaces de glissement, dans des trous oblongs insérés sous forme de trous de glissement de forme librement déterminable ou dans des structures rotatives à pas de denture différent.

2. Écarteur selon la revendication 1, dont le dispositif d'expansion (30) reste à l'intérieur d'un volume délimité par les surfaces d'appui supérieure (21) et inférieure (23) aussi bien à l'état fermé qu'à l'état expansé.

3. Écarteur (10) selon la revendication 1 ou 2, **caractérisé en ce que** la première (21-1, 23-1) ainsi que la deuxième surface partielle (21-2, 23-2) de la surface d'appui supérieure (21) ainsi que de la surface d'appui inférieure (23) présentent, au niveau du bord (24-1, 24-2, 25-1, 25-1) où elles se touchent à l'état fermé, présente une structure emboîtable (26) conçue de telle sorte qu'elle permet, lors d'une expansion, un écartement latéral de la première (21-1, 23-1) et de la deuxième surface partielle (21-2, 23-2) et, à l'état expansé, une fente latérale (27) s'étendant perpendiculairement à la direction de la dérive latérale à travers la surface d'appui supérieure (21) ainsi que la surface d'appui inférieure (23) présentant une largeur de fente (55) qui est inférieure à la valeur de dérive maximale (54) et supérieure ou égale à zéro.

4. Écarteur (10) selon la revendication 3, dont les supports supérieur (20) et inférieur (22) sont formés de telle sorte que, lors d'un écartement latéral, la structure emboîtable (26) de la première surface partielle (21-1, 23-1) glisse reposée sur une structure d'appui (44) dans la partie du support (20, 22) associée à la deuxième surface partielle (21-2, 23-2) et la structure emboîtable (26) de la deuxième surface partielle (21-2, 23-2) glisse reposée sur une structure d'appui (44) dans la partie du support (20, 22) associée à la première surface partielle (21-1, 23-1).

5. Écarteur (10) selon l'une des revendications 1 à 4, dont le dispositif d'expansion (30) destiné à régler l'écarteur (10) entre l'état fermé et l'état expansé comprend une vis sans fin (31) avec une tête de vis (32) permettant de contrôler la modification de l'extension latérale ainsi que de la distance verticale.

6. Écarteur (10) selon la revendication 5, qui ne présente aucune symétrie dans un plan de coupe latéral quelconque le long d'un axe parallèle à l'axe de la vis sans fin (31).

7. Écarteur (10) selon la revendication 5 ou 6, dont le dispositif d'expansion (30) comprend en outre un élément d'écartement mobile (34),
qui est monté dans une position sur l'élément d'écartement (34) de manière à pouvoir pivoter latéralement autour d'un axe mobile (36) et qui peut être déplacé le long de la vis sans fin (31) au moyen d'un écrou fileté (35) intégré dans l'axe mobile (36) et tournant sur un filetage (33) de la vis sans fin (31), l'axe mobile (36) étant disposé de manière à glisser directement ou indirectement dans un trou oblong supérieur (38) dans la première surface partielle (21-1) du support supérieur (20) et dans un trou oblong inférieur (39) dans la première surface partielle (23-1) du support inférieur (22),
- qui peut en outre pivoter latéralement autour d'un axe fixe (37) qui est monté de manière latérale et immobile dans la deuxième surface partielle (21-2) du support supérieur (20) et dans la deuxième surface partielle (23-2) du support inférieur (22), et
- qui est monté de manière à pouvoir glisser librement à une première et/ou à une deuxième extrémité sur une surface de glissement tridimensionnelle supérieure (40) sous la première (21-1) et/ou la deuxième surface partielle (21-2) du support supérieur (20) ainsi que sur une surface de glissement tridimensionnelle inférieure (41) au-dessus de la première (23-1) et/ou la deuxième surface partielle (23-2) du support inférieur (22), la surface de glissement tridimensionnelle supérieure (40) et la surface de glissement tridimensionnelle inférieure (41) étant formées l'une par rapport à l'autre de telle sorte que la première et/ou la deuxième extrémité de l'élément d'écartement (34) occupe, pour chaque angle de rotation de l'élément d'écartement (34) autour de l'axe fixe (37), une position définie sur la surface de glissement tridimensionnelle supérieure (40) ainsi que sur la surface de glissement tridimensionnelle inférieure (41), dont la position absolue et la position correspondante de l'axe mobile (36) dans les trous oblongs de forme correspondante (38, 39), l'extension latérale de l'écarteur (10) et leur distance entre la surface de glissement tridimensionnelle supérieure (40) et la surface de glissement tridimensionnelle inférieure (41) l'une par rapport à l'autre à l'état fermé déterminent la hauteur de l'écarteur (10).

8. Écarteur (10) selon la revendication 5 ou 6, dont le dispositif d'expansion (30) comprend en outre un élément d'écartement mobile (34),
- qui est mobile le long de la vis sans fin (31) et comporte quatre éléments de guidage (42) qui sont chacun logés dans un premier trou oblong supérieur et/ou sous une première surface de glissement de guidage supérieure (38) dans la première surface partielle (21-1) et dans un deuxième trou oblong supérieur et/ou sous une deuxième surface de glissement de guidage supérieure (38) de la deuxième surface partielle (21-2) du support supérieur (20) et dans un premier trou oblong inférieur et/ou sur une première surface de glissement de guidage inférieure (39) de la première surface partielle (23-1) et dans un deuxième trou oblong inférieur et/ou sur une deuxième surface de glissement de guidage inférieure (39) de la deuxième surface partielle (23-2) du support inférieur (22),
- l'élément d'écartement (34) présentant des surfaces de glissement supérieures et inférieures, de préférence tridimensionnelles (40, 41), et
- la surface de glissement supérieure (40) et la surface de glissement inférieure (41) étant formées l'une par rapport à l'autre sur l'élément d'écartement (34), et les trous oblongs et/ou les surfaces de glissement de guidage (38, 39) sont formées et disposées dans chacune des surfaces partielles des supports supérieur et inférieur de telle sorte que, pour une position définie que l'élément d'écartement (34) occupe sur la vis sans fin (31), la position correspondante des éléments de guidage (42) dans les trous oblongs et/ou les surfaces de glissement de guidage (38, 39) de forme correspondante détermine l'extension latérale de l'écarteur (10), et la distance entre la surface de glissement supérieure (40) et la surface de glissement inférieure (41) l'une par rapport à l'autre sur l'élément d'écartement (34) au niveau d'un bord de contact (43) et/ou une position des surfaces de glissement de guidage supérieure (38) et inférieure (39) associée à la position de l'élément d'écartement (34) des supports supérieur (20) et inférieur (22) détermine la hauteur de l'écarteur (10).

9. Écarteur (10) selon la revendication 5 ou 6, dont le dispositif d'expansion (30) comprend en outre une double paire mobile d'éléments d'écartement (34-11, 34-12),
- qui est monté de manière à pouvoir pivoter latéralement autour d'un axe mobile (36-11, 36-12) à un emplacement sur chaque élément d'écartement (34-11, 34-12) de la paire double, les axes mobiles (36-11, 36-12) étant disposés sur un écrou fileté (35) et pouvant glisser le long de la vis sans fin (31) au moyen de l'écrou fileté (35) tournant sur un filetage (33) de la vis sans fin (31),
- le premier élément d'écartement (34-11) de la paire double pouvant pivoter latéralement autour d'un axe fixe (37) qui est monté de manière latérale et immobile dans la première surface partielle (21-1) du support supérieur (20) et dans la première surface partielle (23-1) du support inférieur (22), et le deuxième élément d'écartement (34-12) de la paire double pouvant pivoter latéralement autour d'un axe fixe (37) qui est monté de manière latéralement fixe dans la deuxième surface partielle (21-2) du support supérieur (20) et dans la deuxième surface partielle (23-2) du support inférieur (22), et
- l'écrou fileté (35) qui tourne sur le filetage (33) de la vis sans fin (31) étant conçu de telle sorte qu'il présente des surfaces de glissement (40, 41) ou une double structure à levier coudé (57) vers la première (21-1) et la deuxième surface partielle (21-2) du support supérieur (20) et vers la première (23-1) et la deuxième surface partielle (23-2) du support inférieur (22), qui s'appuient, de manière librement glissant, sur des surfaces de glissement de guidage (38) de la première (21-1) et de la deuxième surface partielle (21-2) du support supérieur (20) et sur des surfaces de glissement de guidage (39) de la première (23-1) et de la deuxième surface partielle (23-2) du support inférieur (22),
- et la position angulaire entre le premier élément d'écartement (34-11) et le deuxième élément d'écartement (34-12) détermine l'extension latérale de l'espaceur (10), et une distance entre la surface de glissement supérieure (40) et la surface de glissement inférieure (41) ou une position de la double structure à levier coudé (57) sur un bord de contact (43) et/ou une position des surfaces de glissement de guidage supérieure (38) et inférieure (39) des supports supérieur (20) et inférieur (22) associée à une position de l'écrou fileté (35) détermine la hauteur de l'espaceur (10).

10. Écarteur (10) selon l'une des revendications 7 à 9, dans lequel la vis sans fin (31) comprend sur sa deuxième moitié (31-2) un filetage (33) qui est opposé à un filetage (33) sur la première moitié (31-1) de la vis sans fin (31), et le dispositif d'expansion (30) comprend un premier élément d'écartement (34-1) ou une première paire double d'éléments d'écartement qui utilise la première moitié (31-1) de la vis sans fin et un deuxième élément d'écartement (34-2) ou une deuxième paire double d'éléments d'écartement qui utilise la deuxième moitié (31-2) de la vis sans fin (31), le premier (34-1) et le deuxième (34-2) élément d'écartement ou la première ou la deuxième double paire d'éléments d'écartement étant associés aux premières (40-1, 41-1) ou deuxièmes (40-2, 41-2) surfaces de glissement tridimensionnelles supérieures et inférieures, et aux trous oblongs supérieurs (38-1, 38-2) et inférieurs (39-1, 39-2) correspondants et/ou aux surfaces de glissement de guidage.

11. Écarteur (10) selon l'une des revendications 7 à 10, comprenant un premier et un deuxième éléments d'écartement ou une double paire d'éléments d'écartement, le premier (34-1) et le deuxième éléments d'écartement (34-2) ou double paire d'éléments d'écartement étant conçus et disposés de telle sorte qu'ils fonctionnent de manière inversée l'un par rapport à l'autre, ou le premier (34-1) et le deuxième élément d'écartement (34-2) ou double paire d'éléments d'écartement étant conçus et disposés de telle sorte qu'ils fonctionnent de manière synchrone l'un par rapport à l'autre.

12. Écarteur (10) selon la revendication 10 ou 11, dont la vis sans fin (31) comporte une structure de guidage (45) entre la première moitié (31-1) et la deuxième moitié (31-2) et/ou entre le premier élément d'écartement (34-1) et le deuxième élément d'écartement (34-2) ou double paires d'éléments d'écartement, qui est montée de manière rotative dans un élément de retenue (46), mais sans pouvoir se déplacer latéralement, l'élément de retenue (46) étant monté de manière mobile, en particulier mobile verticalement, mais sans pouvoir se déplacer latéralement, dans le support supérieur (20) et dans le support inférieur (22).

13. Écarteur (10) selon l'une des revendications 7 à 12, dans lequel la forme et la position des surfaces de glissement tridimensionnelles (40-1, 40-2, 41-2, 41-2) et la forme et la position du trou oblong (38-1, 38-2, 39-1, 39-2) et/ou des surfaces de glissement de guidage sont conçues selon un profil individuel d'expansion requis

14. Écarteur (10) selon l'une des revendications 1 à 13, qui présente une forme réniforme en vue de dessus.

15. Écarteur (10) selon l'une des revendications 1 à 14, qui présente une hauteur minimale (50) supérieure ou égale à 7 mm à l'état fermé et une hauteur maximale (51) inférieure ou égale à 14 mm à l'état expansé, ainsi qu'une extension latérale (52, 53) supérieure ou égale à 13 mm à l'état fermé.
